(19) Europäisches Patentamt
European Patent Office
Office européen des brevets

(11) **EP 4 226 907 A2**

(12) **EUROPÄISCHE PATENTANMELDUNG**

(43) Veröffentlichungstag:
**16.08.2023 Patentblatt 2023/33**

(21) Anmeldenummer: **22214521.1**

(22) Anmeldetag: **19.12.2022**

(51) Internationale Patentklassifikation (IPC):
*A61K 6/62* (2020.01)   *A61K 6/78* (2020.01)
*A61K 6/80* (2020.01)   *A61K 6/833* (2020.01)
*A61K 6/887* (2020.01)   *C08L 33/10* (2006.01)
*C08F 220/12* (2006.01)   *A61K 6/16* (2020.01)
*A61K 6/17* (2020.01)

(52) Gemeinsame Patentklassifikation (CPC):
**C08F 220/12; A61K 6/16; A61K 6/17; A61K 6/62; A61K 6/78; A61K 6/80; A61K 6/833; A61K 6/887; C08L 33/10**

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **22.12.2021 DE 102021134260**

(71) Anmelder: **VOCO GmbH**
**27472 Cuxhaven (DE)**

(72) Erfinder:
• **Willner, Alexander**
**27474 Cuxhaven (DE)**
• **Hahn, Christoph**
**27639 Wurster Nordseeküste (DE)**
• **Plaumann, Manfred Thomas**
**27472 Cuxhaven (DE)**

(74) Vertreter: **Eisenführ Speiser**
**Patentanwälte Rechtsanwälte PartGmbB**
**Postfach 10 60 78**
**28060 Bremen (DE)**

(54) **DENTALE LICHTHÄRTBARE ZUSAMMENSETZUNG SOWIE ENTSPRECHENDE RESTAURATIONEN, HERSTELLVERFAHREN UND VERWENDUNGEN**

(57) Beschrieben wird eine dentale lichthärtbare Zusammensetzung, zum wahlweisen Herstellen einer sichtbaren Restauration im Frontzahnbereich oder zum Füllen einer Kavität im Seitenzahnbereich, umfassend eine Gesamtmenge (A) an radikalisch polymerisierbaren Monomeren, wobei diese Gesamtmenge (A) aus ein, zwei, drei oder mehr als drei radikalisch polymerisierbaren Monomeren besteht und einen Brechungsindex $n_{A,470nm}$ im Bereich von 1,470 bis 1,560 besitzt, eine Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) einen Brechungsindex $n_{B1,\,470nm}$ im Bereich von 1,460 bis 1,570 besitzt und wobei diese Gesamtmenge (B1) im Bereich von 10 bis 30 Volumenprozent liegt, bezogen auf die gesamte dentale lichthärtbare Zusammensetzung, eine Gesamtmenge (B2) an anorganischen Füllstoffpartikeln mit einer Partikelgröße im Bereich von 0,12 $\mu$m bis 10 $\mu$m, wobei diese Gesamtmenge (B2) einen Brechungsindex $n_{B2,\,470nm}$ im Bereich von 1,450 bis 1,560 besitzt, und eine Gesamtmenge (C) an Photoinitiatoren.

EP 4 226 907 A2

**Beschreibung**

Die vorliegende Erfindung betrifft eine dentale lichthärtbare Zusammensetzung, die zum wahlweisen Herstellen einer sichtbaren Restauration im Frontzahnbereich oder zum Füllen einer Kavität im Seitenzahnbereich geeignet ist. Die erfindungsgemäße dentale lichthärtbare Zusammensetzung kann also wahlweise verschiedenen Zwecken dienen, was vom Dentalfachmann bislang als kaum vereinbar angesehen wurde.

Die Erfindung betrifft zudem eine dentale Restauration bzw. ein gehärtetes Dentalmaterial, welche(s) durch Lichthärtung einer erfindungsgemäßen dentalen lichthärtbaren Zusammensetzung erhältlich ist. Die erfindungsgemäße dentale Restauration ist vorzugsweise - und wahlweise - eine sichtbare dentale Restauration im Frontzahnbereich oder eine Füllung einer Kavität im Seitenzahnbereich.

Die vorliegende Erfindung betrifft zudem ein Verfahren zur Herstellung einer erfindungsgemäßen dentalen lichthärtbaren Zusammensetzung.

Die vorliegende Erfindung betrifft auch die Verwendung von Gesamtmengen bestimmter radikalisch polymerisierbarer Monomere und anorganischer Füllstoffpartikel zur Herstellung einer dentalen lichthärtbaren Zusammensetzung. Vorzugsweise ist die hergestellte dentale lichthärtbare Zusammensetzung eine erfindungsgemäße Zusammensetzung.

Die Erfindung betrifft auch eine erfindungsgemäße dentale lichthärtbare Zusammensetzung zur Anwendung in einem Verfahren zur chirurgischen oder therapeutischen Behandlung des menschlichen oder tierischen Körpers und/oder zur Anwendung in einem Diagnostizierverfahren, das am menschlichen oder tierischen Körper vorgenommen wird. Bevorzugt ist dabei eine spezifische Anwendung wahlweise in einem therapeutischen Verfahren zur temporären oder permanenten sichtbaren Restauration im Frontzahnbereich oder in einem therapeutischen Verfahren zum temporären oder permanenten Füllen einer Kavität im Seitenzahnbereich. Weitere bevorzugte spezifische Anwendungen ergeben sich aus den beigefügten Patentansprüchen und der nachfolgenden Beschreibung.

Die Erfindung betrifft auch ein Verfahren zur Herstellung einer sichtbaren Restauration im Frontzahnbereich oder zum Füllen einer Kavität im Seitenzahnbereich.

Die Erfindung wird in den beigefügten Patentansprüchen definiert. Bevorzugte Aspekte der vorliegenden Erfindung ergeben sich überdies aus der nachfolgenden Beschreibung einschließlich der Beispiele.

Soweit für einen erfindungsgemäßen Aspekt (Zusammensetzung; Restauration oder Dentalmaterial; Herstellverfahren; Verwendung; (spezifische) Anwendung in einem Verfahren; Verfahren zur Herstellung einer Restauration oder zum Füllen einer Kavität) bestimmte Ausgestaltungen als bevorzugt bezeichnet werden, gelten die entsprechenden Ausführungen jeweils auch für die anderen Aspekte der vorliegenden Erfindung, *mutatis mutandis.* Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt.

Dentale lichthärtbare Zusammensetzungen sind bereits aus dem Stand der Technik bekannt. Moderne dentale lichthärtbare Zusammensetzungen umfassen dabei häufig eine Menge an radikalisch polymerisierbaren Monomeren, Mikrofüllstoffe (auch Mikrofüller genannt) und Nanofüllstoffe (auch Nanofüller genannt). Weitere Bestandteile moderner dentaler lichthärtbarer Zusammensetzungen sind Photoinitiatoren sowie Farbmittel (beispielsweise Farbpigmente). Entsprechende Zusammensetzungen werden auch als "Nano-Hybrid Composite" bezeichnet. Eine eigene wissenschaftliche Produktinformation zum Nano-Hybrid Composite GrandioSO fasst wesentliche Aspekte und Überlegungen im vorliegenden technischen Bereich zusammen; sie verweist auf übliche Bestimmungsmethoden. Die wissenschaftliche Produktinformation ist im Internet zugänglich: https://www.voco.dental/de/portaldata/1 /resources/products/scientificcompendiums/de/grandioso_scc_de.pdf.

Dentale lichthärtbare Zusammensetzungen sind auch in der Patentliteratur zahlreich offenbart.

EP 2 987 480 A1 (Ivoclar Vivadent AG) offenbart lichthärtende Dentalkomposite mit zunehmender Opazität.

EP 2 902 007 A1 (Tokuyama Dental Corporation) offenbart ein "dental filling repairing material".

EP 3 508 190 A1 offenbart eine "photocurable composition".

EP 3 854 374 A1 und WO 2021/148667 A1 (Ivoclar Vivadent AG) offenbaren ein "ästhetisches dentales Füllungsmaterial mit hoher Durchhärtetiefe".

Die vier vorgenannten Patentdokumente offenbaren jeweils spezifische Brechungsindizes für die eingesetzte Monomermischung bzw. für die verwendeten Füllstoffe.

CN 1 13 730 264 A offenbart ein "Dental gradient color resin ceramic repair material and preparation method thereof".

EP 2 987 480 A1 offenbart, dass die Transluzenz des dort offenbarten Dentalwerkstoffs von den Brechungsindizes der Monomere und der Füllstoffe beeinflusst wird. Die Transluzenz einer (nicht gehärteten) dentalen lichthärtbaren Zusammensetzung ist entscheidend für die Durchhärtetiefe (vgl. erneut EP 2 987 480 A1). Eine große Durchhärtetiefe ist erforderlich, wenn beispielsweise tiefe Kavitäten im Seitenzahnbereich mit einer dentalen lichthärtbaren Zusammensetzung gefüllt werden sollen.

Der Dentalfachmann ist im Lichte des Standes der Technik bestrebt, eine dentale lichthärtbare Zusammensetzung so auszugestalten, dass die Brechungsindizes von Monomeren und Füllstoff nur wenig voneinander abweichen.

Hierbei wurde aber bislang lediglich auf die Brechungsindizes der Makro- bzw. Mikrofüllstoffe geachtet. Hinsichtlich der in modernen dentalen lichthärtbaren Zusammensetzungen vorhandenen Nanofüllstoffe herrschte die Überzeugung, dass deren Brechungsindizes nicht sonderlich relevant sind. So ergibt sich sogar aus der bereits genannten EP 2 902 007 A1, dass Nanofüllstoffe mit einer durchschnittlichen Teilchengröße von weniger als 0,07 μm nicht brauchbar erscheinen. Die Teilchengröße dieser Nanopartikel sei kleiner als die Wellenlänge des sichtbaren Lichts, sodass sie unabhängig von ihrem Brechungsindex transparent erscheinen. Dies soll wiederum dazu führen, dass es schwierig ist, ein gehärtetes Produkt zu erreichen, welches im Aussehen einem natürlichen Zahn entspricht.

Insbesondere für sichtbare Restaurationen im Frontzahnbereich ist es aus ästhetischen Gründen wichtig, dass bereits eine dünne Schicht einer als Restaurationsmaterial eingesetzten ausgehärteten dentalen lichthärtbaren Zusammensetzung nicht mehr übermäßig transluzent ist. Vielmehr ist eine gewisse Opazität erforderlich, damit die dentale Restauration das Aussehen des natürlichen Zahnmaterials möglichst perfekt nachbildet.

Es ergeben sich somit insgesamt für den Dentalfachmann, der einerseits den technischen Teilbereich der sichtbaren Restaurationen im Frontzahnbereich und andererseits den davon separaten technischen Teilbereich der Kavitätenfüllung im Seitenzahnbereich betrachtet, eine Reihe von Anforderungen, die ihm bislang unvereinbar erschienen. Wohl deshalb gibt es bislang keine dentalen lichthärtbaren Zusammensetzungen im Markt, die mit jeweils überzeugendem Erfolg wahlweise zum Herstellen einer sichtbaren Restauration im Frontzahnbereich oder zum Füllen einer Kavität im Seitenzahnbereich eingesetzt werden können. Die besagten und bislang als unvereinbar angenommenen Anforderungen lassen sich wie folgt zusammenfassen:

- Eine dentale lichthärtbare Zusammensetzung zum Herstellen einer sichtbaren Restauration im Frontzahnbereich muss dazu geeignet sein, nach dem Aushärten den ästhetischen Eindruck eines Frontzahns zu erwecken.

- Eine dentale lichthärtbare Zusammensetzung zum Füllen einer tiefen Kavität (zum Beispiel Kavitättiefe > 3 mm) im Seitenzahnbereich muss eine große Durchhärtetiefe besitzen, damit die Zusammensetzung nach dem Einfüllen in die besagte Kavität auch vollständig und in einem Schritt durchgehärtet werden kann. Die dentale lichthärtbare Zusammensetzung muss daher im nicht ausgehärteten Zustand eine entsprechend hohe Transluzenz besitzen. Zu beachten ist insoweit, dass in der dentalen Praxis nahezu ausschließlich eingefärbte (gefärbte) dentale lichthärtbare Zusammensetzungen für praktische Zwecke hergestellt werden, insbesondere bei geplantem Einsatz auch im Frontzahnbereich. Es versteht sich, dass Art und Menge der zur Einfärbung eingesetzten Substanzen Auswirkungen auf die Durchhärtetiefe und die Transluzenz haben. Für die Zwecke fairer Vergleiche sollten daher einerseits ungefärbte dentale lichthärtbare Zusammensetzungen (Basiszusammensetzungen) und auf jeweils identische Weise eingefärbte dentale lichthärtbare Zusammensetzungen miteinander verglichen werden.

- Eine dentale lichthärtbare Zusammensetzung zum Herstellen einer sichtbaren Restauration im Frontzahnbereich und zum Füllen einer Kavität im Seitenzahnbereich muss jeweils hervorragende physikalische und mechanische Eigenschaften aufweisen, wie sie insbesondere durch die Parameter E-Modul (als Maß für die Materialfestigkeit bzw. den Widerstand gegenüber Deformation und Fraktur), ACTA-Abrasion (als Maß für den Substanzverlust durch Reibung) und Polymerisationsschrumpfung (als Maß für die Materialschrumpfung bei Aushärtung) beschrieben werden. Es ist hierbei nicht ausreichend, dass nur einzelne ausgewählte Anforderungen der mechanischen Eigenschaften erfüllt werden. Vielmehr muss eine in der Praxis eingesetzte dentale lichthärtbare Zusammensetzung widerstandsfähig gegenüber mechanischen (Zug-)Belastungen sein (u.a. charakterisiert durch einen Mindestwert des E-Modul), über einen langen Zeitraum Reibungskräften standhalten (u.a. charakterisiert durch einen Maximalwert der ACTA-Abrasion) und zusätzlich eine nur geringe durch die Aushärtung verursachte Schrumpfung aufweisen (u.a. charakterisiert durch einen Maximalwert der Polymerisationsschrumpfung).

Es war eine primäre Aufgabe der vorliegenden Erfindung, eine dentale lichthärtbare Zusammensetzung anzugeben, die trotz der vorstehend geschilderten Einschätzungen und Schwierigkeiten sowohl dazu geeignet ist, eine sichtbare Restauration im Frontzahnbereich zu bilden als auch dazu geeignet ist, eine Kavität im Seitenzahnbereich zu füllen. Die anzugebende dentale lichthärtbare Zusammensetzung sollte somit je nach den Erfordernissen des Einzelfalls wahlweise für den einen oder für den anderen Zweck eingesetzt werden können. Auf diese Weise soll dem behandelnden Zahnarzt die Möglichkeit gegeben werden, mit einer einzelnen dentalen lichthärtbaren Zusammensetzung, zwei unterschiedliche therapeutische Maßnahmen zu ergreifen, für die bislang der Einsatz von zwei unterschiedlichen dentalen lichthärtbaren Zusammensetzungen erforderlich war.

Zudem sollte eine entsprechende dentale Restauration bzw. ein entsprechendes gehärtetes Dentalmaterial angegeben werden, das durch Lichthärtung einer solchen dentalen lichthärtbaren Zusammensetzung erhältlich ist. Hierbei sollte beachtet werden, dass üblicherweise und vorzugsweise die Lichthärtung unter Verwendung von Licht in einem Wellenlängenbereich von 450 nm bis 490 nm stattfindet, das heißt unter Verwendung von blauem Licht.

Zudem sollte ein entsprechendes Verfahren zur Herstellung einer (erfindungsgemäßen) dentalen lichthärtbaren Zusammensetzung angegeben werden. Des Weiteren sollte berücksichtigt werden, dass die anzugebende dentale lichthärtbare Zusammensetzung zur Anwendung in den vorstehend diskutierten chirurgischen bzw. therapeutischen Verfahren geeignet ist, das heißt insbesondere geeignet ist zur spezifischen Anwendung in einem therapeutischen Verfahren zur temporären oder permanenten sichtbaren Restauration im Frontzahnbereich oder in einem therapeutischen Verfahren zum temporären oder permanenten Füllen einer Kavität im Seitenzahnbereich.

Mit Blick auf ein entsprechendes, anzugebendes Verfahren zur Herstellung einer sichtbaren Restauration im Frontzahnbereich oder zum Füllen einer Kavität im Seitenzahnbereich sollte berücksichtigt werden, dass das Aushärten der applizierten dentalen lichthärtbaren Zusammensetzung vorzugsweise durch Bestrahlen mit Licht im Wellenlängenbereich von 450 nm bis 490 nm (blaues Licht) erfolgen sollte.

Die primäre Aufgabe der vorliegenden Erfindung wird gelöst durch eine dentale lichthärtbare Zusammensetzung gemäß dem beigefügten Patentanspruch 1. Die primäre Aufgabe wird somit gelöst durch eine dentale lichthärtbare Zusammensetzung, zum wahlweisen Herstellen einer sichtbaren Restauration im Frontzahnbereich oder zum Füllen einer Kavität im Seitenzahnbereich,

umfassend

- eine Gesamtmenge (A) an radikalisch polymerisierbaren Monomeren, wobei diese Gesamtmenge (A) aus ein, zwei, drei oder mehr als drei radikalisch polymerisierbaren Monomeren besteht und einen Brechungsindex $n_{A,\,470nm}$ im Bereich von 1,470 bis 1,560 besitzt,
- eine Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) einen Brechungsindex $n_{B1,\,470nm}$ im Bereich von 1,460 bis 1,570 besitzt und wobei diese Gesamtmenge (B1) im Bereich von 10 bis 30 Volumenprozent liegt, bezogen auf die gesamte dentale lichthärtbare Zusammensetzung,
- eine Gesamtmenge (B2) an anorganischen Füllstoffpartikeln mit einer Partikelgröße im Bereich von 0,12 $\mu$m bis 10 $\mu$m, wobei diese Gesamtmenge (B2) einen Brechungsindex $n_{B2,470nm}$ im Bereich von 1,450 bis 1,560 besitzt, und
- eine Gesamtmenge (C) an Photoinitiatoren,

wobei für die Brechungsindizes der Gesamtmenge (A) an radikalisch polymerisierbaren Monomeren und der Gesamtmenge (B1) an nicht-aggregierten und nicht-agglomerierten anorganischen Füllstoffpartikeln mit einer Partikelgröße im Bereich von 7 bis 70 nm gilt:

$$n_{B1,\,470nm} - 0,025 < n_{A,\,470nm} < n_{B1,\,470nm} + 0,030$$

und

wobei für die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 $\mu$m bis 10 $\mu$m gilt:

$$n_{B2,\,470nm} - 0,015 < n_{A,\,470nm} < n_{B2,\,470nm} + 0,040$$

und

wobei für die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 $\mu$m bis 10 $\mu$m gilt:

$$n_{B2,\,470nm} - 0,020 < n_{B1,\,470nm} < n_{B2,\,470nm} + 0,035.$$

Die Erfindung basiert auf einer ganzen Reihe ineinandergreifender Überlegungen, die insgesamt dazu führen, dass die vorstehend geschilderten Schwierigkeiten überwunden werden konnten.

Die Erfinder haben zunächst beachtet, dass eine dentale lichthärtbare Zusammensetzung, die zum Füllen einer Kavität im Seitenzahnbereich geeignet sein soll, eine hervorragende Durchhärtetiefe besitzen muss, so wie dies in ähnlicher Weise bereits bei der Konzeption von Dentalkompositen berücksichtigt wurde, die für den Zweck der Erzeugung von Kavitätenfüllungen im Seitenzahnbereich maßgeschneidert wurden.

Die Erfinder haben zudem berücksichtigt, dass die gehärtete Restauration, d. h. die dentale lichthärtbare Zusammensetzung nach der Härtung, üblicherweise nach der Bestrahlung mit blauem Licht, eine nur geringe Transluzenz besitzen darf. Denn nur gehärtete Dentalmaterialien mit einer geringen Transluzenz vermögen den ästhetischen Eindruck eines Frontzahns zu erwecken und sind somit als sichtbare Restauration im Frontzahnbereich geeignet.

Die Erfinder haben des Weiteren berücksichtigt, dass die gehärtete Restauration, d. h. die dentale lichthärtbare Zusammensetzung nach der Härtung, den hohen mechanischen Anforderungen entsprechen muss, die an Dentalmaterialien gestellt werden.

Die genannten Merkmale betreffend die Durchhärtetiefe und die Transluzenz sowie die mechanischen Eigenschaften nach der Härtung spiegeln die äußerst unterschiedlichen Anforderungen wider, welche einerseits beim Füllen einer Kavität im Seitenzahnbereich und andererseits beim Herstellen einer sichtbaren Restauration im Frontzahnbereich zu berücksichtigen sind.

Mit der vorliegenden Erfindung ist es gelungen, alle drei Anforderungen mit einer einzelnen dentalen lichthärtbaren Zusammensetzung zu erfüllen. Hierfür wiederum ist die Zusammenstellung geeigneter Materialien entscheidend, wie sie vorstehend definiert ist.

Erfindungsgemäß umfasst die dentale lichthärtbare Zusammensetzung eine Gesamtmenge (A) an radikalisch polymerisierbaren Monomeren, wobei diese Gesamtmenge (A) aus ein, zwei, drei oder mehr als drei radikalisch polymerisierbaren Monomeren besteht und einen Brechungsindex $n_{A,\,470nm}$ im Bereich von 1,470 bis 1,560 besitzt (weil das bzw. die Monomere entsprechend ausgewählt und mengenmäßig aufeinander abgestimmt sind). Für das Verständnis der vorliegenden Erfindung ist dabei wichtig festzustellen, dass der Brechungsindex wie angegeben bei einer Wellenlänge von 470 nm bestimmt wird, das heißt im Wellenlängenbereich des üblicherweise bei Lichthärtung verwendeten blauen Lichts. Die vorliegende Erfindung berücksichtigt also im Unterschied zu der bislang üblichen Herangehensweise des Dentalfachmanns in besonderem Maße den Umstand, dass die Lichthärtung in der dentalen Praxis regelmäßig unter Verwendung von blauem Licht erfolgt. Es kommt daher für den Erfolg der vorliegenden Erfindung wesentlich darauf an, dass die eingesetzten Materialien und deren Eigenschaften unter Berücksichtigung des Aushärteverfahrens und somit unter Berücksichtigung des üblichen Einsatzes von blauem Licht ausgewählt werden. Für den Fachmann versteht sich, dass jedem Brechungsindex $n_{A,\,470nm}$ (also einem Brechungsindex, der bei einer Wellenlänge von 470 nm, d. h. im Bereich des blauen Lichts bestimmt wurde) auch ein Brechungsindex entspricht, der (zum Beispiel) bei der Wellenlänge von gelbem Licht (zum Beispiel Natrium-D-Linie, 589 nm) bestimmt wird. Die erfindungsgemäße Auswahl der radikalisch polymerisierbaren Monomeren erfolgt aber wie gesagt so, dass die Gesamtmenge (A) den angestrebten Brechungsindex $n_{A,\,470nm}$ besitzt.

Die erfindungsgemäße dentale lichthärtbare Zusammensetzung umfasst neben der Gesamtmenge an radikalisch polymerisierbaren Monomeren zusätzlich die vorstehend definierten Gesamtmengen (B1) und (B2) an anorganischen Füllstoffpartikeln.

Insbesondere umfasst die erfindungsgemäße Zusammensetzung eine Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) einen Brechungsindex $n_{B1,\,470nm}$ im Bereich von 1,460 bis 1,570 besitzt. Entscheidend ist somit wiederum der Brechungsindex der Gesamtmenge (B1) bei einer Messung mit einer Wellenlänge von 470 nm, das heißt im Wellenlängenbereich des blauen Lichts. Im Rahmen der vorliegenden Erfindung wurde festgestellt, dass es für den erfindungsgemäßen Erfolg wesentlich ist, dass für die Brechungsindizes der Gesamtmenge (A) an radikalisch polymerisierbaren Monomeren und die Brechungsindizes der Gesamtmenge (B1) an nicht-aggregierten und nicht-agglomerierten anorganischen Füllstoffpartikeln mit einer Partikelgröße im Bereich von 7 bis 70 nm gilt:

$$n_{B1,\,470nm} - 0{,}025 < n_{A,\,470nm} < n_{B1,\,470nm} + 0{,}030.$$

Mit anderen Worten sollen die (jeweils bei 470 nm bestimmten) Brechungsindizes der Gesamtmenge (A) und der Gesamtmenge (B1) jeweils in einem eng definierten Bereich liegen (1,470 bis 1,560, bzw. 1,460 bis 1,570) und zudem allenfalls geringfügig voneinander abweichen. Eine solche doppelte Berücksichtigung der Brechungsindizes von Nanofüllstoffen mit einer Partikelgröße im Bereich von 7 bis 70 nm ist im Stand der Technik ohne Vorbild.

Mit der vorliegenden Erfindung wird berücksichtigt, dass entgegen den bisherigen Einschätzungen der Fachleute auch der Brechungsindex der besagten Nanofüllstoffe eine relevante Auswirkung auf die Eigenschaften einer dentalen Zusammensetzung vor und nach dem Aushärten hat. Wenngleich die Erfinder der vorliegenden Erfindung noch keine feste wissenschaftliche Begründung für die eigenen experimentellen Erfolge angeben können, gehen sie im Moment doch davon aus, dass die physikalischen Effekte der Rayleigh-Streuung (das heißt die Streuung von Licht an Teilchen oder Partikeln, die kleiner sind als die Wellenlänge des verwendeten Lichts) wie sie bei Bestrahlung von Nanopartikeln auftreten, berücksichtigt werden müssen. Die Rayleigh-Streuung ist dabei stark abhängig von der Frequenz des auf die Partikel auftreffenden Lichts, so wird blaues Licht mit einer höheren Frequenz stärker gestreut als niederfrequentes rotes Licht. Dieser Streuungseffekt muss also insbesondere bei Einsatz von blauem Licht berücksichtigt werden, um die Eigenschaften einer entsprechenden dentalen Zusammensetzung vorhersagen oder gar vorbestimmen zu können. Die Erfinder nehmen an, dass bei Einsatz von blauem Licht an den Nanopartikeln der Gesamtmenge (B1) Streueffekte, und zusätzlich destruktive Interferenzeffekte stattfinden, welche die Durchhärtetiefe in einem Komposit beeinflussen. Um negative Auswirkungen von Nanofüllstoffen auf die Transluzenz (im nicht-ausgehärteten Zustand) und somit auf die Durchhärtetiefe zu reduzieren oder zu vermeiden, wird erfindungsgemäß vorgesehen, dass die Gesamtmenge (B1) an nicht-aggregierten und nicht-agglomerierten anorganischen (Nano-)Füllstoffpartikeln hinsichtlich ihres Brechungsindex spezifisch ausgewählt wird und - wie vorstehend bereits ausgeführt - an den Brechungsindex der Gesamtmenge (A) der radikalisch polymerisierbaren Monomeren angepasst ist, um eine möglichst hohe Homogenität der Brechungsindizes innerhalb der erfindungsgemäßen dentalen lichthärtbaren Zusammensetzung einzustellen. Auf diesem Wege wird erfindungsgemäß eine hohe Durchhärtetiefe, bevorzugt eine Durchhärtetiefe von 3,5 mm oder mehr, gewährleistest, natürlich im Zusammenspiel mit den weiteren Bestandteilen der erfindungsgemäßen Zusammensetzung.

Die erfindungsgemäße dentale lichthärtbare Zusammensetzung umfasst neben der Gesamtmenge (A) an radikalisch polymerisierbaren Monomeren (wie vorstehend ausgeführt) und der Gesamtmenge (B1) an (Nano-)Füllstoffpartikeln (wie vorstehend ausgeführt) eine Gesamtmenge (B2) an anorganischen Füllstoffpartikeln mit einer Partikelgröße im Bereich von 0,12 $\mu$m bis 10 $\mu$m, wobei diese Gesamtmenge (B2) einen Brechungsindex $n_{B2,470nm}$ im Bereich von 1,450 bis 1,560 besitzt. Hierbei handelt es sich um Mikrofüllstoffe, deren (jeweils bei 470 nm bestimmten) Brechungsindizes $n_{B2,\,470nm}$ an die vorstehend diskutierten Brechungsindizes der Gesamtmenge (A) an radikalisch polymerisierbaren Monomeren und der Gesamtmenge (B1) an Nanofüllstoffen angepasst ist. Insbesondere gilt für die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 $\mu$m bis 10 $\mu$m:

$$n_{B2,\,470nm} - 0{,}015 < n_{A,\,470nm} < n_{B2,\,470nm} + 0{,}040.$$

Erfindungsgemäß werden also nicht lediglich pauschal die Brechungsindizes von Monomeren einerseits und Füllstoffpartikeln (insgesamt) andererseits zueinander in Beziehung gesetzt und in geeigneter Weise ausgewählt, vielmehr wird hinsichtlich der Füllstoffpartikel präzise zwischen den Brechungsindizes der Gesamtmenge (B1) an (Nano-)Füllstoffpartikeln (wie vorstehend weiter definiert) und der Gesamtmenge (B2) an (Mikro-)Füllstoffpartikeln (wie vorstehend weiter definiert) unterschieden.

Mit der vorliegenden Erfindung wird weiterhin berücksichtigt, dass auch die Brechungsindizes der Gesamtmenge (B1) an (Nano-)Füllstoffpartikeln (wie vorstehend weiter definiert) und der Gesamtmenge (B2) an (Mikro-)Füllstoffpartikeln (wie vorstehend weiter definiert) aneinander angepasst sind. Insbesondere gilt für die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 $\mu$m bis 10 $\mu$m:

$$n_{B2,\,470nm} - 0{,}020 < n_{B1,\,470nm} < n_{B2,\,470nm} + 0{,}035$$

Diese Abstimmung der Brechungsindizes der partikulären Hauptbestandteile der dentalen lichthärtbaren Zusammensetzung, d.h. der Nano- und der Mikrofüllstoffpartikel der Gesamtmengen (B1) und (B2), trägt zu einer hohen Homogenität der Brechungsindizes innerhalb der erfindungsgemäßen dentalen lichthärtbaren Zusammensetzung bei und gewährleistet somit (auch bei hochgefüllten Dentalkompositen) eine hohe Durchhärtetiefe.

Erfindungsgemäß werden also sämtliche Brechungsindizes der Gesamtmengen (A), (B1) und (B2) betrachtet und so ausgewählt und aufeinander abgestimmt, dass bei der Lichthärtung von erfindungsgemäßen dentalen lichthärtbaren Zusammensetzungen unter Verwendung von Licht in einem Wellenlängenbereich von 450 nm bis 490 nm (blaues Licht) eine hohe Durchhärtetiefe gewährleistet wird. Eine solche dreifache Abstimmung der Brechungsindizes von i) radikalisch polymerisierbaren Monomeren, ii) Nanofüllstoffpartikeln und iii) Mikrofüllstoffpartikeln ist im Stand der Technik ohne Vorbild.

Wie oben und in den beigefügten Patentansprüchen definiert, umfasst die erfindungsgemäße dentale lichthärtbare Zusammensetzung eine Gesamtmenge (B1) an nicht-aggregierten und nicht-agglomerierten anorganischen Füllstoffpartikeln mit einer Partikelgröße im Bereich von 7 bis 70 nm (Nanofüllstoffpartikel), wobei diese Gesamtmenge (B1) im Bereich von 10 bis 30 Volumenprozent liegt, bezogen auf die gesamte dentale lichthärtbare Zusammensetzung.

Es hat sich gezeigt, dass bei Einsatz eines hohen Volumenanteils an Nanofüllstoffpartikeln von mindestens 10 Volumenprozent nach der Härtung der erfindungsgemäßen Zusammensetzung hervorragende physikalische und mechanische Eigenschaften erreicht werden. Es hat sich auch gezeigt, dass ein hoher Anteil an Nanofüllstoffpartikeln von mehr als 30 Volumenprozent die mechanischen Eigenschaften in vielen Fällen in nachteiliger Weise beeinflusst.

Es ist den Erfindern nicht nur gelungen, Zusammensetzungen zu identifizieren, die den ästhetischen Eindruck eines Frontzahns erwecken (insbesondere ist die erreichte Transluzenz nach dem Aushärten hervorragend) und eine große Durchhärtetiefe besitzen. Bevorzugte erfindungsgemäße Zusammensetzungen besitzen nämlich vorzugsweise die folgenden mechanischen Eigenschaften, bestimmt mit den nachfolgend angegebenen jeweiligen Bestimmungsmethoden:

- ein Elastizitätsmodul, bestimmt anhand der Steigung im linear-elastischen Bereich des Graphen aus dem Spannungs-Dehnungs-Diagramm der Biegefestigkeitsmessung durch Herstellung und Aushärtung von Prüfkörpern mit den Dimensionen $2 \times 2 \times 25$ mm analog zu dem in der ISO 4049:2019 beschriebenen Verfahren, von mehr als 10 GPa,

  bevorzugt von mehr als 11,5 GPa,
  besonders bevorzugt von mehr als 13 GPa,
  und/oder (bevorzugt "und")

  - eine ACTA-Abrasion, bestimmt nach Aushärtung mittels einer Verschleißsimulation nach ISO/TS 14569 2, von weniger als 70 $\mu$m,

  bevorzugt von weniger als 60 $\mu$m,
  besonders bevorzugt von weniger als 55 $\mu$m,
  und/oder (bevorzugt "und")

  - eine Polymerisationsschrumpfung, bestimmt mittels der bonded-disc-Methode und eine Stunde nach der Belichtung von Prüfkörpern mit einem Lichthärtegerät mit einem Strahlungsflussmaximum bei einer Wellenlänge im Bereich von 440 nm bis 490 nm bei einer Temperatur von 23 °C und für einen Zeitraum von 40 s, von weniger als 2,0 %,

  bevorzugt von weniger als 1,8 %,
  besonders bevorzugt von weniger als 1,6 %.

Der Begriff "Gesamtmenge (A) an radikalisch polymerisierbaren Monomeren" bezeichnet die Gesamtmenge sämtlicher in der erfindungsgemäßen dentalen lichthärtbaren Zusammensetzung enthaltenen radikalisch polymerisierbaren Monomere. Neben dieser Gesamtmenge (A) gibt es also keine weitere Menge an radikalisch polymerisierbaren Monomeren in der erfindungsgemäßen Zusammensetzung.

Die "Gesamtmenge (A) an radikalisch polymerisierbaren Monomeren" umfasst vorzugsweise ein, zwei oder mehr Monomere ausgewählt aus der Gruppe bestehend aus 2-Hydroxyethyl(meth)acrylat, 2-Hydroxypropyl(meth)acrylat, 3-Hydroxypropyl(meth)acrylat, 1,2-Dihydroxypropyl(meth)acrylat, 1,3-Dihydroxypropyl(meth)acrylat, Ethylenglykol-di(meth)acrylat, Diethylenglykoldi(meth)acrylat, Triethylenglykoldi(meth)acrylat, Tetraethy-lenglykoldi(meth)acrylat, Polyethylenglykoldi(meth)acrylat, Propylenglykoldi(meth)acrylat, Dipropylenglykoldi(meth)acrylat, Tripropylenglykoldi(meth)acrylat, Tetrapropylenglykol-di(meth)acrylat, Polypropylenglykoldi(meth)acrylat, 1,4-Butandioldi(meth)acrylat, 1,3-Bu-tandioldi(meth)acrylat, 1,6-Hexandioldi(meth)acrylat, 1,10-Decandioldi(meth)acrylat, 1,12-Dodecandioldi(meth)acrylat, 2-Hydroxypropyl-1,3-di(meth)acrylat, 3-Hydroxypropyl-1,2-di(meth)acrylat, 2,2-Bis[4-[3-(meth)acryloyloxy2-hydroxypropoxy]phenyl]propan, 2,2-Bis[4-(meth)acryloyloxyphenyl]-propan, 2,2-Bis[4-(meth)acryloyloxyethoxyphenyl]propan, 2,2-Bis[4-(meth)-acryloyloxydiethoxyphenyl]propan, 2,2-Bis[4-(meth)acryloyloxytriethoxyphe-nyl]-propan 2,2-Bis[4-(meth)acryloyloxytetraethoxyphenyl]propan, 2,2-Bis[4-(meth)-acry-loyloxypentaethoxyphenyl]-propan, 2,2-Bis[4-(meth)acryloyloxydipropoxy-phenyl]propan, 2,2-Bis[4-(meth)acryloyloxyethoxyphenyl]-2-[4-(meth)acryloyl-oxydiethoxyphenyl]propan, 2-[4-(Meth)acryloyloxydiethoxyphenyl]-2-[4-(meth)-acryloyloxytriethoxyphenyl]propan, 2-[4-(Meth)acryloyloxdipropoxyphenyl]-2-[4-(meth)acryloyloxytriethoxyphenyl]propan, 2,2-Bis[4-(meth)acryloyloxyisopropoxy phe-nyl]propan, 7,7,9-Trimethyl-3,14-dioxa-4,13-dioxo-5,12-diazahexadecan-1,16-dioxydi(meth)acrylat, 7,9,9-Trimethyl-3,14-dioxa-4,13-dioxo-5,12-diazahexadecan-1,16-dioxydi(meth)acrylat, Neopentylglykoldi(meth)acrylat, Trime-thylolpropantri(meth)acrylat, Pentaerythritoldi(meth)acrylat, 3(4),8(9)-Bis((meth)acryloy-loxymethyl)tricyclo[5.2.1.0$^{2,6}$]decan und Dimethacrylate bzw. Diacrylate des Dihydroxymethyltricyclo[5.2.1.0$^{2,6}$]decans. Vorzugsweise sind sämtliche in der erfindungsgemäßen Zusammensetzung enthaltenen radikalisch polymerisierbaren Monomeren aus der genannten Gruppe ausgewählt.

In einigen Fällen besonders bevorzugt und daher in der erfindungsgemäßen Zusammensetzung enthalten sind Dimethacrylate bzw. Diacrylate des Dihydroxymethyl-tricyclo[5.2.1.0$^{2,6}$]decans, wie sie z.B. in den Druckschriften DE 2419887 A1, DE 2406557 A1, DE 2931926 A1, DE 3522005 A1, DE 3522006 A1, DE 3703120 A1, DE 102005021332 A1, DE 102005053775 A1, DE 102006060983 A1, DE 69935794 T2 und DE 102007034457 A1 beschrieben werden.

Ganz besonders bevorzugt und daher in der erfindungsgemäßen Zusammensetzung enthalten sind radikalisch polymerisierbare Monomere ausgewählt aus der Gruppe bestehend aus 2,2-Bis[4-[3-(meth)acryloyloxy2-hydroxypropoxy]phenyl]propan, 2,2-Bis[4-(meth)acry-loyloxyphenyl]-propan, 2,2-Bis[4-(meth)acryloyloxyethoxyphenyl]propan, 2,2-Bis[4-(meth)-acryloyloxydiethoxyphenyl]propan, 2,2-Bis[4-(meth)acryloyloxytriethoxyphenyl]-propan 2,2-Bis[4-(meth)acryloyloxytetraethoxyphenyl]propan, 2,2-Bis[4-(meth)-acryloyloxypen-taethoxyphenyl]-propan, 2,2-Bis[4-(meth)acryloyloxydipropoxy-phenyl]propan, 2,2-Bis[4-(meth)acryloyloxyethoxyphenyl]-2-[4-(meth)acryloyl-oxydiethoxyphenyl]propan, 2-[4-(Meth)acryloyloxydiethoxyphenyl]-2-[4-(meth)-acryloyloxytriethoxyphenyl]propan, 2-[4-(Meth)acryloyloxdipropoxyphenyl]-2-[4-(meth)acryloyloxytriethoxyphenyl]propan, 2,2-Bis[4-(meth)acryloyloxyisopropoxy phe-nyl]propan, 7,7,9-Trimethyl-3,14-dioxa-4,13-dioxo-5,12-diazahexadecan-1,16-dioxydi(meth)acrylat, 7,9,9-Trimethyl-3,14-dioxa-4,13-dioxo-5,12-diazahexadecan-1,16-dioxydi(meth)acrylat und 3(4),8(9)-Bis((meth)acryloyloxyme-thyl)tricyclo[5.2.1.0$^{2,6}$]decan.

Der Begriff "Gesamtmenge (B1) an nicht-aggregierten und nicht-agglomerierten anorganischen Füllstoffpartikeln mit einer Partikelgröße im Bereich von 7 bis 70 nm", bezeichnet die Gesamtmenge sämtlicher Füllstoffpartikel in der erfindungsgemäßen Zusammensetzung, die nicht-aggregiert, nicht-agglomeriert und anorganisch sind und dabei eine Partikelgröße im Bereich von 7 bis 70 nm besitzen. Eine weitere Menge solcher nicht-aggregierten und nicht-agglomerierten anorganischen Füllstoffpartikel mit einer Partikelgröße im Bereich von 7 bis 70 nm existiert somit in der erfindungsgemäßen dentalen lichthärtbaren Zusammensetzung nicht.

Der Begriff "Gesamtmenge (B2) an anorganischen Füllstoffpartikeln mit einer Partikelgröße im Bereich von 0,12 $\mu$m bis 10 $\mu$m" bezeichnet in entsprechender Weise die Gesamtmenge sämtlicher anorganischer Füllstoffpartikel mit einer Partikelgröße im Bereich von 0,12 $\mu$m bis 10 $\mu$m in der erfindungsgemäßen Zusammensetzung. Eine weitere Menge an anorganischen Füllstoffpartikeln mit einer Partikelgröße im Bereich von 0,12 $\mu$m bis 10 $\mu$m existiert somit in der erfindungsgemäßen dentalen lichthärtbaren Zusammensetzung nicht.

Die "Gesamtmenge (B2) an anorganischen Füllstoffpartikeln mit einer Partikelgröße im Bereich von 0,12 $\mu$m bis 10 $\mu$m" umfasst vorzugsweise ein, zwei oder mehr Materialien ausgewählt aus der Gruppe bestehend aus Bariumsilikatgläser, Bariumfluorsilikatgläser, Strontiumsilikatgläser, Strontiumfluorsilikatgläser, Bariumalumniumsilikatgläser, Bariumborosilikatgläser, Bariumfluoroaluminosilikatgläser, Fluoraluminiumsilikatgläser, Ytterbiumfluorid, Feldspat und amorphen Materialien auf Basis der Oxide oder Mischoxide von Silicium, Aluminium, Zirconium und/oder Titan. Vorzugsweise sind sämtliche in der erfindungsgemäßen Zusammensetzung enthaltenen anorganischen Füllstoffpartikel mit einer Partikelgröße im Bereich von 0,12 $\mu$m bis 10 $\mu$m aus der genannten Gruppe ausgewählt.

Für die drei diskutierten Gesamtmengen (A), (B1) und (B2) gilt jeweils, dass es keine entsprechende weitere Gesamtmenge oder Teilmenge gibt, die (jeweils unter Beachtung der stofflichen Charakteristik) einen Brechungsindex außerhalb des jeweils angegebenen Bereiches besitzt.

Der Begriff "Gesamtmenge (C) an Photoinitiatoren" bezeichnet die Gesamtmenge sämtlicher Photoinitiatoren in der erfindungsgemäßen Zusammensetzung. Photoinitiatoren zur Lichthärtung von radikalisch polymerisierbaren Monomeren sind dem Fachmann bekannt. Bevorzugte Photoinitiatoren der Gesamtmenge (C) sind ausgewählt aus der Gruppe bestehend aus Campherchinon (CQ) und Ethyl-4-(dimethylamino)benzoat (DABE). Besonders bevorzugt sind erfindungsgemäße Zusammensetzungen, die als Photoinitiatoren sowohl Campherchinon (CQ) als auch Ethyl-4-(dimethylamino)benzoat (DABE) enthalten.

Bevorzugt ist eine erfindungsgemäße dentale lichthärtbare Zusammensetzung, wobei die Gesamtmenge (A) an radikalisch polymerisierbaren Monomeren, die Gesamtmenge (B1) an nicht-aggregierten und nicht-agglomerierten anorganischen Füllstoffpartikeln mit einer Partikelgröße im Bereich von 7 bis 70 nm, die Gesamtmenge (B2) an anorganischen Füllstoffpartikeln mit einer Partikelgröße im Bereich von 0,12 $\mu$m bis 10 $\mu$m und die Gesamtmenge (C) an Photoinitiatoren so ausgewählt ist, dass

die dentale lichthärtbare Zusammensetzung
eine Durchhärtetiefe von 3,5 mm oder mehr besitzt, bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$,

und/oder (bevorzugt "und")

die dentale lichthärtbare Zusammensetzung nach der Härtung
eine Transluzenz von weniger als 45 % besitzt, bevorzugt eine Transluzenz von weniger als 40 % besitzt, besonders bevorzugt eine Transluzenz von weniger als 35 % besitzt.

Wie vorstehend und in den beigefügten Patentansprüchen definiert, besitzt eine bevorzugte erfindungsgemäße dentale lichthärtbare Zusammensetzung eine Durchhärtetiefe (Polymerisationstiefe) von 3,5 mm oder mehr, bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$. Der Fachmann ist mit der Bestimmung der Durchhärtetiefe dentaler lichthärtbarer Zusammensetzungen vertraut. Sofern nicht im Einzelfall anders angegeben, beziehen sich im Rahmen des vorliegenden Textes im Zusammenhang mit der vorliegenden Erfindung stehende Angaben für eine Durchhärtetiefe bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$ auf Messungen gemäß ISO 4049:2019.

Wie oben und in den beigefügten Patentansprüchen angegeben, besitzt eine bevorzugte erfindungsgemäße dentale lichthärtbare Zusammensetzung nach der Härtung eine Transluzenz von weniger als 45 %, bevorzugt eine Transluzenz von weniger als 40 %, besonders bevorzugt eine Transluzenz von weniger als 35 %. Derartig geringe Transluzenzen sind für Restaurationen im sichtbaren Frontzahnbereich besonders bevorzugt. Transluzenzmessungen sind dem Fachmann vertraut. Soweit nicht im Einzelfall anders angegeben, beziehen sich im Zusammenhang mit der vorliegenden Erfindung angegebene Messwerte auf Messungen gemäß der unten angegebenen Messmethode.

Bevorzugt ist eine erfindungsgemäße dentale lichthärtbare Zusammensetzung, wobei für die Brechungsindizes der Gesamtmenge (A) an radikalisch polymerisierbaren Monomeren und der Gesamtmenge (B1) an nicht-aggregierten und nicht-agglomerierten anorganischen Füllstoffpartikeln mit einer Partikelgröße im Bereich von 7 bis 70 nm gilt:

$$n_{B1,\,470nm} - 0{,}020 < n_{A,\,470nm} < n_{B1,\,470nm} + 0{,}025,$$

$$\text{bevorzugt } n_{B1,\,470nm} - 0{,}015 < n_{A,\,470nm} < n_{B1,\,470nm} + 0{,}020,$$

und/oder (vorzugsweise "und")

wobei für die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 $\mu$m bis 10 $\mu$m gilt:

$$n_{B2,\,470nm} - 0{,}010 < n_{A,\,470nm} < n_{B2,\,470nm} + 0{,}035,$$

$$\text{bevorzugt } n_{B2,\,470nm} - 0{,}005 < n_{A,\,470nm} < n_{B2,\,470nm} + 0{,}030,$$

und/oder (vorzugsweise "und")
wobei für die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 $\mu$m bis 10 $\mu$m gilt:

$$n_{B2,\,470nm} - 0{,}015 < n_{B1,\,470nm} < n_{B2,\,470nm} + 0{,}030,$$

$$\text{bevorzugt } n_{B2,\,470nm} - 0{,}010 < n_{B1,\,470nm} < n_{B2,\,470nm} + 0{,}025.$$

Gemäß den vorstehenden Ausführungen ist es besonders bevorzugt, wenn die Brechungsindizes (gemessen bei 470 nm, d. h. im Wellenlängenbereich des blauen Lichts) der Gesamtmenge (A) an radikalisch polymerisierbaren Monomeren und der Gesamtmenge (B1) an nicht-aggregierten und nicht-agglomerierten anorganischen Füllstoffpartikeln mit einer Partikelgröße im Bereich von 7 bis 70 nm besonders nahe beieinanderliegen. Hieraus resultiert bei Einsatz von blauem Licht eine besonders hohe Transluzenz und damit eine besonders große Durchhärtetiefe. Derartig hohe Durchhärtetiefen von dentalen lichthärtbaren Zusammensetzungen sind zum Füllen einer Kavität im Seitenzahnbereich besonders bevorzugt. Es hat sich gezeigt, dass die Gesamtmenge (B1) an nicht-aggregierten und nicht-agglomerierten anorganischen Füllstoffpartikeln mit einer Partikelgröße im Bereich von 7 bis 70 nm einen Brechungsindex besitzen sollte, der möglichst identisch ist mit dem Brechungsindex der Gesamtmenge (A) an radikalisch polymerisierbaren Monomeren.

Wie vorstehend ausgeführt ist es gleichermaßen besonders bevorzugt, wenn die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 $\mu$m bis 10 $\mu$m besonders eng beieinanderliegen. Auch insoweit hat sich gezeigt, dass die jeweiligen Brechungsindizes möglichst identisch sein sollten.

Es ist gleichermaßen besonders bevorzugt, wenn die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 $\mu$m bis 10 $\mu$m besonders eng beieinanderliegen. Es hat sich auch hier gezeigt, dass die jeweiligen Brechungsindizes der Nano- und Mikrofüllstoffpartikel möglichst identisch sein sollten.

Vorzugsweise sind die vorstehend diskutierten bevorzugten Ausgestaltungen miteinander kombiniert, da so gleichzeitig eine besonders geringe Transluzenz nach Aushärtung und besonders große Durchhärtetiefen erreicht werden.

Bevorzugt ist eine erfindungsgemäße dentale lichthärtbare Zusammensetzung (vorzugsweise wie vorstehend oder nachfolgend als bevorzugt bezeichnet), wobei

- die Gesamtmenge (A) an radikalisch polymerisierbaren Monomeren,

im Bereich von 21 bis 34 Volumenprozent liegt, bezogen auf die gesamte dentale lichthärtbare Zusammensetzung

und/oder

im Bereich von 6 bis 20 Gewichtsprozent liegt, bezogen auf die gesamte dentale lichthärtbare Zusammensetzung

und/oder (vorzugsweise "und")

- die Gesamtmenge (B1) an nicht-aggregierten und nicht-agglomerierten anorganischen Füllstoffpartikeln mit einer Partikelgröße im Bereich von 7 bis 70 nm, bevorzugt die Gesamtmenge (B1-p1) an nicht-aggregierten und nicht-agglomerierten anorganischen Füllstoffpartikeln mit einer Partikelgröße im Bereich von 7 bis 60 nm, besonders bevorzugt die Gesamtmenge (B1-p2) an nicht-aggregierten und nicht-agglomerierten anorganischen Füllstoffpartikeln mit einer Partikelgröße im Bereich von 7 bis 50 nm,

im Bereich von 13 bis 27 Volumenprozent liegt, bevorzugt im Bereich von 15 bis 25 Volumenprozent, bezogen auf die gesamte dentale lichthärtbare Zusammensetzung

und/oder

im Bereich von 10 bis 66 Gewichtsprozent liegt, bevorzugt im Bereich von 13 bis 63 Gewichtsprozent, besonders bevorzugt im Bereich von 14 bis 60 Gewichtsprozent, bezogen auf die gesamte dentale lichthärtbare Zusammensetzung

und/oder (vorzugsweise "und")

- die Gesamtmenge (B2) an anorganischen Füllstoffpartikeln mit einer Partikelgröße im Bereich von 0,12 $\mu$m bis 10 $\mu$m, bevorzugt die Gesamtmenge (B2-p1) an anorganischen Füllstoffpartikeln mit einer Partikelgröße im Bereich von 0,18 $\mu$m bis 10 $\mu$m, besonders bevorzugt die Gesamtmenge (B2-p2) an anorganischen Füllstoffpartikeln mit einer Partikelgröße im Bereich von 0,40 $\mu$m bis 10 $\mu$m,

im Bereich von 42 bis 60 Volumenprozent liegt, bezogen auf die gesamte dentale lichthärtbare Zusammensetzung

und/oder

im Bereich von 28 bis 70 Gewichtsprozent liegt, bezogen auf die gesamte dentale lichthärtbare Zusammensetzung.

Bevorzugt sind Ausgestaltungen, in denen die besagten Gesamtmengen (A), (B1) und (B2) gemeinsam den weit überwiegenden Anteil an der erfindungsgemäßen dentalen lichthärtbaren Zusammensetzung ausmachen.

Vorzugsweise addieren sich die Gesamtmengen (A), (B1) und (B2) einer erfindungsgemä-$\beta$en dentalen lichthärtbaren Zusammensetzung (vorzugsweise einer vorstehend als bevorzugt bezeichneten erfindungsgemäßen dentalen lichthärtbaren Zusammensetzung) auf zumindest 95 Volumenprozent, vorzugsweise zumindest 98 Volumenprozent, bezogen auf die gesamte dentale lichthärtbare Zusammensetzung (d. h. deren Gesamtvolumen) und/oder auf zumindest 95 Gewichtsprozent, vorzugsweise 98 Gewichtsprozent, bezogen auf die gesamte dentale lichthärtbare Zusammensetzung (d. h. deren Gesamtmasse).

Ebenfalls vorzugsweise enthält eine erfindungsgemäße dentale lichthärtbare Zusammensetzung (vorzugsweise eine vorstehend als bevorzugt bezeichnete erfindungsgemäße dentale lichthärtbare Zusammensetzung) einen Anteil von kleiner als 5 Gewichtsprozent, vorzugsweise von kleiner als 3 Gewichtsprozent, an Kompositfüllstoffen, jeweils bezogen auf die gesamte dentale lichthärtbare Zusammensetzung (d. h. deren Gesamtmasse).

Unter "Kompositfüllstoffen" werden insoweit organische Polymerpartikel verstanden, die ihrerseits mit anorganischen Füllstoffen bzw. Füllstoffpartikeln gefüllt sind. Dies entspricht der Definition aus WO 2021/148667 A1.

Besonders bevorzugt ist eine erfindungsgemäße dentale lichthärtbare Zusammensetzung (vorzugsweise eine vorstehend als bevorzugt bezeichnete erfindungsgemäße dentale lichthärtbare Zusammensetzung) die keine Kompositfüllstoffe enthält.

Gemäß den vorstehenden Ausführungen ist es besonders bevorzugt, wenn erfindungsgemäße dentale lichthärtbare Zusammensetzungen einen hohen Volumen- und/oder Massenanteil der Gesamtmengen (A), (B1) und (B2) aufweisen (wie vorstehend beschrieben). Überraschend hat sich gezeigt, dass in erfindungsgemäßen dentalen lichthärtbaren Zusammensetzungen auf die im Stand der Technik in größeren Mengen eingesetzten Kompositfüllstoffe verzichtet werden kann, vgl. z.B. die Dentalwerkstoffe gemäß WO 2021/148667 A1 und EP 3 854 374 A1 mit einem Anteil von bis zu 60 Gew.-% an Kompositfüllstoffen. Insbesondere durch die gezielte Abstimmung der jeweiligen Volumen- und/oder Massenanteile der Gesamtmengen (A), (B1) und (B2) zueinander werden auch bei Abwesenheit von Kompositfüllstoffen (oder anderen Füllstoffen, die nicht unter die Definition der Gesamtmengen (A), (B1) und (B2) fallen) hervorragende mechanische Eigenschaften wie E-Modul, ACTA-Abrasion und Polymerisationsschrumpfung der ausgehärteten Dentalkomposite erreicht (wie vorstehend und nachstehend beschrieben). In Bezug auf mechanische Eigenschaften wie E-Modul, ACTA-Abrasion und Polymerisationsschrumpfung der ausgehärteten Dentalkomposite ist es besonders vorteilhaft, wenn die bevorzugten erfindungsgemä-ßen dentalen lichthärtbaren Zusammensetzungen einen vergleichsweise hohen Anteil an Nanofüllstoffpartikeln der Gesamtmenge (B1) von mindestens 13 und bevorzugt mindestens 15 Volumenprozent aufweisen, bezogen auf die gesamte dentale lichthärtbare Zusammensetzung.

Erfindungsgemäß ist der Volumenanteil an Nanofüllstoffpartikeln der Gesamtmenge (B1) nach oben begrenzt, da sich die dentalen lichthärtbaren Zusammensetzungen bei einem zu hohen Feststoffanteil häufig nur mit besonderem Aufwand verarbeiten lassen.

Bevorzugt ist eine erfindungsgemäße dentale lichthärtbare Zusammensetzung (vorzugsweise wie vorstehend oder nachfolgend als bevorzugt bezeichnet), wobei die Gesamtmenge (B1) an nicht-aggregierten und nicht-agglomerierten anorganischen Füllstoffpartikeln mit einer Partikelgröße im Bereich von 7 bis 70 nm, bevorzugt die Gesamtmenge (B1-p1) an nicht-aggregierten und nicht-agglomerierten anorganischen Füllstoffpartikeln mit einer Partikelgröße im Bereich von 7 bis 60 nm, besonders bevorzugt die Gesamtmenge (B1-p2) an nicht-aggregierten und nicht-agglomerierten anorganischen Füllstoffpartikeln mit einer Partikelgröße im Bereich von 7 bis 50 nm, einen Anteil von größer als 31 Gewichtsprozent ausmacht, jeweils bezogen auf die gesamte dentale lichthärtbare Zusammensetzung (d.h. deren Gesamtmasse).

Erfindungsgemäß ist dabei der Massenanteil an Nanofüllstoffpartikeln der Gesamtmenge (B1) nach oben begrenzt, da sich die dentalen lichthärtbaren Zusammensetzungen bei einem zu hohen Feststoffanteil häufig nur mit besonderem Aufwand verarbeiten lassen.

Der Fachmann berücksichtigt bei der Auswahl des Volumenanteils und/oder des Massenanteils der Gesamtmenge (B1) sowohl das angestrebte vorteilhafte mechanische Eigenschaftsprofil der ausgehärteten Dentalkomposite als auch die für das Herstellen einer sichtbaren Restauration im Frontzahnbereich oder zum Füllen einer Kavität im Seitenzahnbereich benötigte Fließfähigkeit der dentalen lichthärtbaren Zusammensetzung.

Entsprechende Überlegungen gelten für die Gesamtmenge (B2); insoweit ist ein Anteil der Gesamtmenge (B2) von mindestens 42 Volumenprozent, bezogen auf die gesamte dentale lichthärtbare Zusammensetzung, besonders vorteilhaft zum Erreichen vorteilhafter mechanischer Eigenschaften des ausgehärteten Dentalkomposits.

Der Unterschied zwischen den bevorzugten Volumenprozentbereichen und Gewichtsprozentbereichen der Gesamtmenge (B1) ist begründet durch die materialspezifischen Dichten der anorganischen Füllstoffpartikel. So hat Ytterbiumfluorid mit 8,2 g/cm$^3$ eine etwa viermal höhere Dichte als SiO$_2$ mit 2,2 g/cm$^3$. Entscheidend für die vorteilhaften mechanischen Eigenschaften ist hierbei der volumenprozentuale Anteil an Nanofüllstoffpartikeln der Gesamtmenge (B1).

Bevorzugt ist eine erfindungsgemäße dentale lichthärtbare Zusammensetzung (vorzugsweise wie vorstehend oder nachfolgend als bevorzugt bezeichnet), wobei die Gesamtmenge (B1) an nicht-aggregierten und nicht-agglomerierten anorganischen Füllstoffpartikeln mit einer Partikelgröße im Bereich von 7 bis 70 nm aus Füllstoffpartikeln besteht, die (i) eine gleiche oder unterschiedliche stoffliche Zusammensetzung besitzen und (ii) zusätzlich oberflächenmodifiziert sind oder nicht oberflächenmodifiziert sind, wobei die Füllstoffpartikel vorzugsweise (i) eine gleiche stoffliche Zusammensetzung besitzen und (ii) zusätzlich oberflächenmodifiziert sind.

Hierbei besteht die Gesamtmenge (B1) vorzugsweise

- aus einer Menge von Füllstoffpartikeln gleicher stofflicher Zusammensetzung, welche einen Brechungsindex

$n_{B1, 470nm}$ im Bereich von 1,460 bis 1,570 besitzt, wobei in dieser Menge die Füllstoffpartikel zusätzlich oberflächenmodifiziert sind oder nicht oberflächenmodifiziert sind,

oder

- aus einer Mischung von zwei, drei oder mehr als drei Teilmengen von Füllstoffpartikeln, wobei die Füllstoffpartikel innerhalb einer jeden Teilmenge (i) eine gleiche stoffliche Zusammensetzung besitzen und (ii) zusätzlich oberflächenmodifiziert sind oder nicht oberflächenmodifiziert sind, aber die Füllstoffpartikel aus unterschiedlichen Teilmengen eine unterschiedliche stoffliche Zusammensetzung besitzen, wobei jede dieser Teilmengen für sich betrachtet einen Brechungsindex $n_{B1, 470nm}$ im Bereich von 1,460 bis 1,570 besitzt, wobei vorzugsweise die Differenz zwischen dem höchsten Brechungsindex und dem niedrigsten Brechungsindex der jeweils für sich betrachteten Teilmengen kleiner ist als 0,01.

Für die vorstehende Definition bevorzugter Füllstoffpartikel der Gesamtmenge (B1) wird unterschieden zwischen der (i) stofflichen Zusammensetzung der Füllstoffpartikel ohne Berücksichtigung einer etwaigen Oberflächenmodifikation und der (ii) gegebenenfalls zusätzlich vorhandenen Oberflächenmodifikation. So können erste und zweite Füllstoffpartikel innerhalb der Gesamtmenge (B1) eine gleiche stoffliche Zusammensetzung besitzen, wobei die ersten Füllstoffpartikel zusätzlich oberflächenmodifiziert sind und die zweiten Füllstoffpartikel (bei gleicher stofflicher Zusammensetzung) nicht oberflächenmodifiziert sind. Zudem kann es beispielsweise Füllstoffpartikel der Gesamtmenge (B1) geben, die eine unterschiedliche stoffliche Zusammensetzung besitzen, aber jeweils oberflächenmodifiziert sind.

Vorhandene Oberflächenmodifikationen können dabei gleich oder unterschiedlich sein.

Vorzugsweise besitzen jedoch - wie vorstehend ausgeführt - die Füllstoffpartikel der Gesamtmenge (B1) eine gleiche stoffliche Zusammensetzung und sind zusätzlich (in gleicher oder unterschiedlicher Weise) oberflächenmodifiziert.

Besonders bevorzugte, nicht-aggregierte und nicht-agglomerierte anorganische Füllstoffpartikel mit einer Partikelgröße im Bereich von 7 bis 70 nm umfassen oder bestehen aus Ytterbiumtrifluorid ($YbF_3$) oder Siliziumoxid ($SiO_z$). Dabei sind die bevorzugten Partikel aus Ytterbiumtrifluorid ($YbF_3$) vorzugsweise oberflächenmodifiziert, und zwar besonders bevorzugt mit MDP oberflächenmodifiziert. MDP bezeichnet dabei 10-[(2-Methylprop-2-enoyl)oxy]decyl dihydrogen phosphat (CAS-Nr. 85590-00-7). Die bevorzugten Füllstoffpartikel aus Siliziumoxid ($SiO_z$) sind vorzugsweise oberflächenmodifiziert, bevorzugt oberflächenmodifiziert mit MPS, wobei MPS [3-(Methacryloyloxy)propyl]trimethoxysilan bedeutet (CAS-Nr. 2530-85-0). Die vorstehend genannte "stoffliche Zusammensetzung" wird also charakterisiert durch "Ytterbiumtrifluorid" bzw. "Siliziumoxid," die Oberflächenmodifikation wird durch Angabe der zur Oberflächenmodifikation des jeweiligen anorganischen Materials eingesetzten chemischen Verbindung charakterisiert. Entsprechende bevorzugte Füllstoffpartikel können auch in Kombination miteinander eingesetzt werden.

Bevorzugt ist eine erfindungsgemäße dentale lichthärtbare Zusammensetzung (vorzugsweise wie vorstehend oder nachfolgend als bevorzugt bezeichnet), wobei die Gesamtmenge (B2-p2) an anorganischen Füllstoffpartikeln mit einer Partikelgröße im Bereich von 0,40 $\mu$m bis 10 $\mu$m, bevorzugt die Gesamtmenge (B2-p1) an anorganischen Füllstoffpartikeln mit einer Partikelgröße im Bereich von 0,18 $\mu$m bis 10 $\mu$m, besonders bevorzugt die

Gesamtmenge (B2) an anorganischen Füllstoffpartikeln mit einer Partikelgröße im Bereich von 0,12 $\mu$m bis 10 $\mu$m

- aus einer Menge von Füllstoffpartikeln gleicher stofflicher Zusammensetzung besteht, die vorzugsweise zusätzlich oberflächenmodifiziert sind, wobei diese Menge von Füllstoffpartikeln einen Brechungsindex $n_{B2,470nm}$ im Bereich von 1,450 bis 1,560 besitzt,

oder

- aus einer Mischung von zwei, drei oder mehr als drei Teilmengen von Füllstoffpartikeln besteht, wobei die Füllstoffpartikel innerhalb einer jeden Teilmenge eine gleiche stoffliche Zusammensetzung besitzen und vorzugsweise zusätzlich oberflächenmodifiziert sind, aber die Füllstoffpartikel aus unterschiedlichen Teilmengen eine unterschiedliche stoffliche Zusammensetzung besitzen, wobei jede dieser Teilmengen für sich betrachtet einen Brechungsindex $n_{B2,470nm}$ im Bereich von 1,450 bis 1,560 besitzt, wobei vorzugsweise die Differenz zwischen dem höchsten Brechungsindex und dem niedrigsten Brechungsindex der jeweils für sich betrachteten Teilmengen kleiner ist als 0,01.

Die im Zusammenhang mit bevorzugten Ausgestaltungen der anorganischen Füllstoffpartikel der Gesamtmenge (B1) hinsichtlich der Begriffe "stoffliche Zusammensetzung" und "oberflächenmodifiziert" gemachten Ausführungen gelten für die Gesamtmenge (B2) entsprechend.

In eigenen Experimenten hat sich gezeigt, dass bei Einsatz einer Mischung von zwei, drei oder mehr als drei Teilmengen von Füllstoffpartikeln darauf zu achten ist, dass der Brechungsindex jeder einzelnen Teilmenge vorzugsweise für sich betrachtet die für die Gesamtmenge (B2) angegebenen Eigenschaften besitzt und dass die Differenz der Brechungsindizes der Teilmengen möglichst gering ist. Auf diese Weise wird eine besonders große Transluzenz der nichtgehärteten Zusammensetzung und damit eine besonders große Durchhärtetiefe erreicht.

Bevorzugt ist eine erfindungsgemäße dentale lichthärtbare Zusammensetzung (vorzugsweise wie vorstehend oder nachfolgend als bevorzugt bezeichnet), wobei die Gesamtmenge (A) an radikalisch polymerisierbaren Monomeren so ausgewählt ist, dass durch ihre Lichthärtung ein polymeres Material erhältlich ist, welches einen um mindestens 0,015, vorzugsweise mindestens 0,020, erhöhten Brechungsindex besitzt, bestimmt bei einer Wellenlänge von 589 nm.

Die hier genannte Wellenlänge von 589 nm liegt im Bereich des gelben Lichts. Es handelt sich um die Wellenlänge der dem Fachmann bekannten Natrium-D-Linie. Verglichen werden somit der Brechungsindex der Gesamtmenge (A) an radikalisch polymerisierbaren Monomeren vor dem Aushärten und nach dem Aushärten der dentalen lichthärtbaren Zusammensetzung, wobei jeweils unter Einsatz von Licht der Wellenlänge 589 nm gemessen wird. Diese Wellenlänge liegt innerhalb des grün-gelben Bereichs des Lichtspektrums (500 nm - 600 nm), welcher vom menschlichen Auge besonders intensiv wahrgenommen wird und somit bei der Betrachtung einer sichtbaren Restauration im Frontzahnbereich relevant ist und maßgeblich zum ästhetischen Gesamteindruck beiträgt. Die Erfindung berücksichtigt, dass der Brechungsindex der Gesamtmenge (A) an radikalisch polymerisierbaren Monomeren vor der Aushärtung deutlich niedriger ist, als der Brechungsindex des daraus resultierenden polymeren Materials. Die Änderung dieses Brechungsindex (bestimmt bei einer Wellenlänge von 589 nm) ist entscheidend dafür, dass das ausgehärtete Dentalmaterial für den Betrachter opak (d. h. weniger transluzent) erscheint, obwohl die erfindungsgemäße dentale lichthärtbare Zusammensetzung vor dem Aushärten eine besonders hohe Transluzenz besaß. Die erhöhte Opazität (verringerte Transluzenz) nach Lichthärtung wird erreicht, weil eine Brechungsindex-Erhöhung (ausgehend von den Monomeren hin zum Polymer) stattfindet, ohne dass gleichzeitig die Brechungsindizes der Füllstoffe variieren (denn die Füllstoffpartikel verändern sich ja bei der Lichthärtung der Monomere stofflich nicht).

Nach der Lichthärtung resultiert eine Brechungsindex-Differenz zwischen dem ausgehärteten Polymer einerseits und den darin eingebetteten Füllstoffpartikeln andererseits. Diese Brechungsindex-Differenz ist in an sich bekannter Weise verantwortlich für die erhöhte Opazität (verringerte Transluzenz).

Bevorzugt ist eine erfindungsgemäße dentale lichthärtbare Zusammensetzung (vorzugsweise wie vorstehend oder nachfolgend als bevorzugt bezeichnet), zusätzlich zu den Gesamtmengen (A), (B1), (B2) und (C) umfassend eine Gesamtmenge (D) an Farbmitteln ausgewählt aus der Gruppe bestehend aus anorganischen Farbpigmenten, organischen Farbpigmenten und Farbstoffen.

Die Farbmittel der Gesamtmenge (D) sind bevorzugt ausgewählt aus der Gruppe bestehend aus Eisenoxid, Titaniumdioxid, Bariumsulfat und Aluminiumoxid.

Bevorzugt ist eine derartige erfindungsgemäße dentale lichthärtbare Zusammensetzung, die also zusätzlich eine Gesamtmenge (D) an Farbmitteln umfasst,

wobei

- für die Transluzenz $T_{Col}$ dieser dentalen lichthärtbaren Zusammensetzung nach der Härtung im Vergleich mit der Transluzenz $T_{NoCol}$ einer ansonsten identisch zusammengesetzten dentalen lichthärtbaren Zusammensetzung, die keine zusätzlichen Farbmittel umfasst, gilt:

$$35\ \% > T_{NoCol} - T_{Col} > 5\ \%,\ \text{bevorzugt}\ 30\ \% > T_{NoCol} - T_{Col} > 5\ \%,\ \text{besonders bevorzugt}\ 25\ \% > T_{NoCol} - T_{Col} > 5\ \%,$$

und/oder (vorzugsweise "und")

wobei

- die Gesamtmenge (D) an dem einen oder den mehreren Farbmitteln

maximal einen Volumenanteil von 800 ppm bildet, bevorzugt maximal 600 ppm, besonders bevorzugt maximal 400 ppm, bezogen auf die gesamte dentale lichthärtbare Zusammensetzung,

und/oder

maximal einen Massenanteil von 2000 ppm bildet, bevorzugt maximal 1500 ppm, besonders bevorzugt maximal 1000 ppm, bezogen auf die gesamte dentale lichthärtbare Zusammensetzung,

und/oder (vorzugsweise "und")

wobei die dentale lichthärtbare Zusammensetzung nach der Härtung eine Transluzenz $T_{Col}$ von weniger als 28 % besitzt, bevorzugt von weniger als 26 %, besonders bevorzugt von weniger als 24 %, insbesondere bevorzugt von weniger als 22 %.

Die Transluzenz $T_{NoCol}$ der ansonsten identisch zusammengesetzten dentalen lichthärtbaren Zusammensetzung, die keine zusätzlichen Farbmittel umfasst, ist höher als die Transluzenz $T_{Col}$ der bevorzugten dentalen lichthärtbaren Zusammensetzung, die eine Gesamtmenge (D) an Farbmitteln umfasst. Die Differenz der beiden genannten Transluzenzen ist bevorzugt größer als 5 %. Die Transluzenz wird jeweils nach der Härtung bestimmt, sodass deutlich wird, dass der Zusatz an Farbmitteln einen bevorzugten Beitrag zum ästhetischen Gesamteindruck beispielsweise von dentalen Restaurationen im sichtbaren Frontzahnbereich leistet, indem er die Transluzenz in gewünschter Weise reduziert. Die Reduktion der Transluzenz soll aber andererseits auch nicht übertrieben groß sein, da sonst ebenfalls kein ästhetischer Eindruck entstehen würde, der einem natürlichen Zahn entspricht. Der durch den Zusatz von Farbmitteln in einer Gesamtmenge (D) erreichte Effekt hinsichtlich der Reduktion der Transluzenz addiert sich zu den weiter oben diskutierten Effekten, die auf der Brechungsindex-Änderung des Kunststoffmaterials basieren (die radikalisch polymerisierbaren Monomere der Gesamtmenge (A) reagieren durch Lichthärtung zu einem ausgehärteten Polymer mit erhöhtem Brechungsindex).

Wie vorstehend ausgeführt ist der Anteil der zusätzlichen Farbmittel der Gesamtmenge (D) in der erfindungsgemäßen dentalen lichthärtbaren Zusammensetzung vorzugsweise gering (siehe die vorstehenden Angaben zu bevorzugten maximalen Volumenanteilen und bevorzugten maximalen Massenanteilen).

Es ist ein besonderer Vorteil der vorliegenden Erfindung, dass bereits bei Einsatz geringer Mengen an Farbmitteln eine gewünschte geringe Transluzenz der dentalen lichthärtbaren Zusammensetzung nach der Härtung erreicht wird. Dies liegt natürlich an dem bereits vorstehend diskutierten Effekt der Brechungsindex-Erhöhung des Kunststoffmaterials durch die Polymerisation der eingesetzten Monomere. Der geringe Anteil an Farbmitteln, der zum Erreichen der erforderlichen Transluzenz nach dem Härten benötigt wird, führt dazu, dass die Durchhärtetiefe (vor dem Härten) der erfindungsgemäßen dentalen lichthärtbaren Zusammensetzung auch bei Anwesenheit von Farbmitteln besonders hoch bleibt, im Unterschied zu Zusammensetzungen aus dem Stand der Technik.

Nach der Härtung besitzt eine bevorzugte erfindungsgemäße dentale lichthärtbare Zusammensetzung, die zusätzliche Farbmittel enthält, eine Transluzenz $T_{Col}$ von weniger als 28 %, bevorzugt von weniger als 26 %, besonders bevorzugt von weniger als 24 %, insbesondere bevorzugt von weniger als 22 %.

Bevorzugt ist eine erfindungsgemäße dentale lichthärtbare Zusammensetzung (vorzugsweise wie vorstehend oder nachfolgend als bevorzugt bezeichnet), wobei in der Zusammensetzung die Gesamtmenge sämtlicher anorganischer Partikel mit einer Partikelgröße von maximal 0,12 $\mu$m eine durchschnittliche volumenbezogene Partikelgröße von weniger als 70 nm besitzt, bestimmt mittels dynamischer Lichtstreuung, vorzugsweise eine durchschnittliche volumenbezogene Partikelgröße von weniger als 66 nm.

Im Stand der Technik bestand die Einschätzung, dass anorganische Nanopartikel mit einer durchschnittlichen Partikelgröße von weniger als 70 nm nachteilig sind. So wird beispielsweise in EP 2 902 007 A1 gelehrt, dass eingesetzte anorganische Füllstoffe eine durchschnittliche Partikelgröße von nicht weniger als 0,07 $\mu$m besitzen sollten.

Im Rahmen der vorliegenden Erfindung ist jedoch der Einsatz von derartigen besonders kleinen Partikelgrößen besonders bevorzugt. Wichtig ist dabei jedoch, dass die Brechungsindizes der eingesetzten Materialien so ausgewählt sind, wie es vorstehend definiert ist.

Bevorzugt ist eine erfindungsgemäße dentale lichthärtbare Zusammensetzung (vorzugsweise wie vorstehend oder nachfolgend als bevorzugt bezeichnet), wobei für die Farbwerte der dentalen lichthärtbaren Zusammensetzung nach der Härtung, bestimmt gemäß EN ISO 11664-4, gilt:

55,0 < L* < 80,0, bevorzugt 64,0 < L* < 75,0, und

-1,5 < a* < 4,5, bevorzugt 2,0 < a* < 4,5, und

5,0 < b* < 20,0, bevorzugt 13,0 < b* < 20,0,
wobei bevorzugt gilt:

64,0 < L* < 75,0, und

2,0 < a* < 4,5, und

13,0 < b* < 20,0.

Die Farbe der dentalen lichthärtbaren Zusammensetzung kann dabei durch entsprechenden Einsatz geeigneter Farbmittel eingestellt werden. Die vorstehend angegebenen bevorzugten Farbwerte der dentalen lichthärtbaren Zusammensetzung nach der Härtung entsprechen der Farbe typischer natürlicher Zahnsubstanzen.

Bevorzugt ist eine erfindungsgemäße dentale lichthärtbare Zusammensetzung (vorzugsweise wie vorstehend oder nachfolgend als bevorzugt bezeichnet), die zusätzlich zu den Gesamtmengen (A), (B1), (B2) und (C) eine Gesamtmenge (E) an ein, zwei, drei oder mehr als drei Hilfsstoffen umfasst, bei denen es sich nicht um Farbmittel handelt, wobei die Hilfsstoffe der Gesamtmenge (E) ausgewählt sind aus der Gruppe bestehend aus:

- rheologische Hilfsmittel,

- Polymerisationsinitiatoren, die keine Photoinitiatoren sind,

- chemische Verbindungen als Katalysatoren bzw. Bestandteile von Katalysatorsystemen,

- Stabilisatoren, insbesondere UV- und Tageslichtstabilisatoren,

- Inhibitoren,

- Aktivatoren,

- Molekulargewichtsregler,

- Konservierungsmittel,

- grenzflächenaktive Substanzen,

- Biozide, vorzugsweise Bakterizide,

- organische Polymere und Oligomere und Verbindungen mit hohen Molekulargewichten, vorzugsweise Weichmacher,

- Verdickungsmittel, und

- dentale Arzneimittel.

Bevorzugte erfindungsgemäße dentale lichthärtbare Zusammensetzungen (wie vorstehend definiert, vorzugsweise wie vorstehend als bevorzugt definiert), enthalten vorzugsweise einen oder mehrere Inhibitoren (auch Stabilisatoren genannt). Diese werden üblicherweise zugesetzt, um eine Spontanpolymerisation zu vermeiden. Das erhöht die Lagerstabilität der bevorzugten dentalen lichthärtbaren Zusammensetzungen. Bevorzugt einzusetzende Inhibitoren sind Phenolderivate wie Hydrochinonmonomethylether (MeHQ) oder 2,6-Di-tert.butyl-4-methylphenol (BHT). Weitere bevorzugt einzusetzende Inhibitoren wie tert.-Butylhydroxyanisol (BHA), 2,2-Diphenyl-1-picrylhydrazyl-, Galvinoxyl-, Triphenylmethyl-Radikale, 2,2,6,6,-Tetra-methylpiperidinyl-1-oxylradikale (TEMPO) sowie Derivate von TEMPO oder Phenothiazin sowie Derivate dieser Verbindung werden in EP 0 783 880 B1 beschrieben. Alternative bevorzugte Inhibitoren sind in DE 101 19 831 A1 und in EP 1 563 821 A1 angegeben.

Bevorzugt eingesetzte Molekulargewichtsregler sind beispielsweise Aldehyde und Ketone, wie Formaldehyd, Acetaldehyd, Propionaldehyd, n-Butyraldehyd, Isobutyraldehyd, Methylethylketon, Aceton, Methylisobutylketon, Ameisensäure, Ammoniumformiat, Hydroxylammoniumsulfat und Hydroxylammoniumphosphat, Verbindungen, die Schwefel in organisch gebundener Form enthalten, wie Di-n-butylsulfid, Di-n-octylsulfid, Diphenylsulfid, Diisopropyldisulfid, Di-n-butyldisulfid, Di-n-hexyldisulfid, Diacetyldisulfid und Di-tert.-butyl-trisulfid, Verbindungen, die Schwefel in Form von SH-Gruppen aufweisen, wie n-Butylmercaptan, n-Hexylmercaptan und n-Dodecylmercaptan, Octadecylmercaptan, weitere Schwefelverbindungen wie Hydrogensulfite, Disulfite, Verbindungen wie Mercaptoethanol, Mercaptobutanol, Mercaptoessigsäure, 3-Mercaptopropionsäure, Mercaptobernsteinsäure, Thioglycerin, Thioglykolsäure, Diethanolsulfid, Thiodiglykol, Ethylthioethanol, 2,2,4,6,6-Pentamethylheptan-4-thiol, 2,2,4,6,6,8,8-Heptamethylnonan-4-thiol, Thioharnstoff, Dimethylsulfoxid, Ethylhexylthioglycolat, Pentaerythrittetrathioglycolat, Mercaptopropyltrimethoxysilan, dann Allylverbindungen wie Allylalkohol, Allylbromid, oder Benzylverbindungen wie Benzylchlorid oder Alkylhalogenide wie Chloroform, Bromtrichlormethan oder Tetrachlormethan, Tetrabrommethan, Methylenchlorid, weiterhin niedere und höhermolekulare einwertige oder mehrwertige Alkohole wie Methanol, Ethanol, n-Propanol, Isopropanol, tert.-Butanol, sec-Butanol, n-Butanol, Amylalkohol, Cyclohexanol, Octanol, Dodecanol, 1-Ethylhexanol, Glycerin, Stearylalkohol, Oleylalkohol, Hydroxyethylmethacrylat oder Amine wie Triethylamin sowie Toluol oder Ethylbenzol.

Weitere, und in vielen Fällen bevorzugte, Molekulargewichtsregler sind beispielsweise diverse Terpene, insbesondere Terpinene α-Terpinen, β-Terpinen, γ-Terpinen), Phellandrene (α-Phellandren, β-Phellandren) und Terpinolen (auch δ-Terpinen genannt), 1,4-Cyclohexadien (gegebenenfalls substituiert), 1,3-Cyclohexadien (gegebenenfalls substituiert), 1,4-Dihydronaphtalin, 1,4,5,8-Tetrahydronaphthalin, 2,5-Dihydrofuran oder dimeres α-Styrol (2,4-Diphenyl-4-methyl-1-penten) sowie Linolsäure und α-Linolensäure.

Die vorliegende Erfindung betrifft auch eine dentale Restauration und ein gehärtetes Dentalmaterial. Erfindungsgemäß ist eine dentale Restauration oder ein gehärtetes Dentalmaterial, vorzugsweise eine sichtbare dentale Restauration im Frontzahnbereich oder eine Füllung einer Kavität im Seitenzahnbereich, erhältlich durch Lichthärtung einer erfindungsgemäßen dentalen lichthärtbaren Zusammensetzung (vorzugsweise wie vorstehend als bevorzugt bezeichnet), vorzugsweise unter Verwendung von Licht in einem Wellenlängenbereich von 450 nm bis 490 nm.

Die vorstehenden Ausführungen zu bevorzugten erfindungsgemäßen dentalen lichthärtbaren Zusammensetzungen gelten hier in vollem Umfang entsprechend. Wie bereits ausgeführt, ist es dabei vorteilhaft, Merkmale bevorzugter Ausgestaltungen einer erfindungsgemäßen dentalen lichthärtbaren Zusammensetzung miteinander zu kombinieren.

Die Erfindung betrifft auch ein Verfahren zur Herstellung einer (vorzugsweise erfindungsgemäßen, bevorzugt wie vorstehend als bevorzugt bezeichnet) dentalen lichthärtbaren Zusammensetzung, umfassend die folgenden Schritte:

(i) Auswahl einer Gesamtmenge (A) an radikalisch polymerisierbaren Monomeren, einer Gesamtmenge (B1) an nicht-aggregierten und nicht-agglomerierten anorganischen Füllstoffpartikeln mit einer Partikelgröße im Bereich von 7 bis 70 nm, einer Gesamtmenge (B2) an anorganischen Füllstoffpartikeln mit einer Partikelgröße im Bereich von 0,12 $\mu$m bis 10 $\mu$m und einer Gesamtmenge (C) an Photoinitiatoren,
wobei folgende Kriterien zur Auswahl der Gesamtmengen (A), (B1) und (B2) gelten, d. h. vom Fachmann bei Durchführung des Verfahrens beachtet und umgesetzt werden:

- die Gesamtmenge (A) an radikalisch polymerisierbaren Monomeren besitzt einen Brechungsindex $n_{A,\,470nm}$ im Bereich von 1,470 bis 1,560,

- die Gesamtmenge (B1) an nicht-aggregierten und nicht-agglomerierten anorganischen Füllstoffpartikeln mit einer Partikelgröße im Bereich von 7 bis 70 nm besitzt einen Brechungsindex $n_{B1,\,470nm}$ im Bereich von 1,460 bis 1,570,

- die Gesamtmenge (B2) an anorganischen Füllstoffpartikeln mit einer Partikelgröße im Bereich von 0,12 $\mu$m bis 10 $\mu$m besitzt einen Brechungsindex $n_{B2,470nm}$ im Bereich von 1,450 bis 1,560,

- für die Brechungsindizes der Gesamtmenge (A) an radikalisch polymerisierbaren Monomeren und der Gesamtmenge (B1) an nicht-aggregierten und nicht-agglomerierten anorganischen Füllstoffpartikeln mit einer Partikelgröße im Bereich von 7 bis 70 nm gilt:

$$n_{B1,\,470nm} - 0,025 < n_{A,\,470nm} < n_{B1,\,470nm} + 0,030,$$

- für die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 $\mu$m bis 10 $\mu$m gilt:

$$n_{B2,\,470nm} - 0,015 < n_{A,\,470nm} < n_{B2,\,470nm} + 0,040,$$

- für die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 $\mu$m bis 10 $\mu$m gilt:

$$n_{B2,\,470nm} - 0,020 < n_{B1,\,470nm} < n_{B2,\,470nm} + 0,035,$$

(ii) Herstellen oder Bereitstellen der ausgewählten Gesamtmengen (A), (B1), (B2) und (C),

(iii) Mischen der gemäß Schritt (ii) hergestellten bzw. bereitgestellten Gesamtmengen (A), (B1), (B2) und (C), sowie gegebenenfalls zusätzlich einer Gesamtmenge (D) von Farbmitteln und/oder einer Gesamtmenge (E) an ein, zwei, drei oder mehr als drei zusätzlichen Hilfsstoffen, so dass die dentale lichthärtbare Zusammensetzung resultiert.

Es versteht sich, dass jede einzelne der im erfindungsgemäßen Verfahren eingesetzten Gesamtmengen und Substanzen für sich betrachtet bereits aus dem Stand der Technik bekannt sein kann. Es ist aber eine besondere Leistung der vorliegenden Erfindung, dass die besagten Substanzen und deren Gesamtmengen so ausgewählt und eingestellt werden, dass eine dentale lichthärtbare Zusammensetzung resultiert, welche sowohl zum Herstellen einer sichtbaren Restauration im Frontzahnbereich als auch zum Füllen einer Kavität im Seitenzahnbereich geeignet ist. Bei geeigneter Auswahl ergibt sich nämlich eine erfindungsgemäße dentale lichthärtbare Zusammensetzung mit den vorstehend bereits genannten herausragenden Eigenschaften, nämlich einer hohen Durchhärtetiefe, bevorzugt einer Durchhärtetiefe von 3,5 mm oder mehr, bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$ und gleichzeitig mit einer geringen Transluzenz nach der Härtung, bevorzugt einer Transluzenz von weniger als 45 %.

Es ist dabei eine besondere Leistung der Erfinder der vorliegenden Erfindung, dass die Auswahl auf Basis von Brechungsindizes erfolgt, die bei einer Wellenlänge von 470 nm bestimmt wurde, d.h. unter Einsatz eines spezifischen blauen Lichts, wie es in der dentalen Praxis auch zum Aushärten eingesetzt wird.

Die Erfindung betrifft auch die Verwendung einer, zweier oder sämtlicher (vorzugsweise sämtlicher) der folgenden Gesamtmengen:

- Gesamtmenge (A) an radikalisch polymerisierbaren Monomeren, wobei diese Gesamtmenge (A) aus ein, zwei, drei oder mehr als drei radikalisch polymerisierbaren Monomeren besteht und einen Brechungsindex $n_{A,\,470nm}$ im Bereich von 1,470 bis 1,560 besitzt,

- Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) einen Brechungsindex $n_{B1,\,470nm}$ im Bereich von 1,460 bis 1,570 besitzt,

- Gesamtmenge (B2) an anorganischen Füllstoffpartikeln mit einer Partikelgröße im Bereich von 0,12 $\mu$m bis 10 $\mu$m, wobei diese Gesamtmenge (B2) einen Brechungsindex $n_{B2,\,470nm}$ im Bereich von 1,450 bis 1,560 besitzt,
  zur Herstellung einer dentalen lichthärtbaren Zusammensetzung (vorzugsweise einer erfindungsgemäßen dentalen lichthärtbaren Zusammensetzung wie vorstehend definiert und vorzugsweise wie vorstehend als bevorzugt bezeichnet), die geeignet ist zum wahlweisen Herstellen einer sichtbaren Restauration im Frontzahnbereich oder zum Füllen einer Kavität im Seitenzahnbereich, wobei die dentale lichthärtbare Zusammensetzung

- eine Durchhärtetiefe von 3,5 mm oder mehr besitzt, bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$,
  und/oder (vorzugsweise "und")

- nach der Härtung eine Transluzenz von weniger als 45 % besitzt.

Sowohl für das erfindungsgemäße Verfahren zur Herstellung einer dentalen lichthärtbaren Zusammensetzung als auch für die erfindungsgemäße Verwendung einer, zweier oder sämtlicher der angegebenen Gesamtmengen gilt selbstverständlich, dass die im Zusammenhang mit der erfindungsgemäßen dentalen lichthärtbaren Zusammensetzung angegebenen bevorzugten Ausgestaltungen entsprechend zutreffen; erneut gilt, dass Kombinationen von Merkmalen derartiger bevorzugter Ausgestaltungen ebenfalls bevorzugt sind.

Die Erfindung betrifft auch eine erfindungsgemäße dentale lichthärtbare Zusammensetzung zur Anwendung in einem Verfahren zur chirurgischen oder therapeutischen Behandlung des menschlichen oder tierischen Körpers und/oder zur Anwendung in einem Diagnostizierverfahren, das am menschlichen oder tierischen Körper vorgenommen wird,
vorzugsweise zur spezifischen Anwendung

- in einem therapeutischen Verfahren zur temporären oder permanenten sichtbaren Restauration im Frontzahnbereich oder in einem therapeutischen Verfahren zum temporären oder permanenten Füllen einer Kavität im Seitenzahnbereich,
  oder

- in einem therapeutischen Verfahren als

  - Zahnfüllungsmaterial,

- Dentalzement,

- dentales Unterfüllungsmaterial,

- als fließfähiges Kompositmaterial (Flow-Material),

- als Kronenmaterial,

- als Inlay,

- als Onlay,

- als Brückenmaterial

- und/oder als Stumpfaufbaumaterial.

Die Erfindung betrifft selbstverständlich auch die entsprechenden spezifischen Verfahren selbst. Die vorstehenden Anmerkungen zu bevorzugten Ausgestaltungen erfindungsgemä-ßer dentaler lichthärtbarer Zusammensetzungen, etc. gelten auch hier entsprechend.

Die vorliegende Erfindung betrifft somit auch ein Verfahren zur Herstellung einer sichtbaren Restauration im Frontzahnbereich oder zum Füllen einer Kavität im Seitenzahnbereich umfassend die folgenden Schritte:

(i) Herstellen oder Bereitstellen einer erfindungsgemäßen dentalen lichthärtbaren Zusammensetzung (vorzugsweise wie vorstehend als bevorzugt bezeichnet),

(ii) Applizieren der hergestellten oder bereitgestellten dentalen lichthärtbaren Zusammensetzung auf die zu restaurierende oder zu füllende Zahnpartie,

(iii) Aushärten der applizierten dentalen lichthärtbaren Zusammensetzung durch Bestrahlen mit Licht im Wellenlängenbereich von 450 nm bis 490 nm.

Auch insoweit gelten natürlich die vorstehenden Erläuterungen zu bevorzugten erfindungsgemäßen dentalen lichthärtbaren Zusammensetzungen.

Die Erfindung wird nachfolgend anhand von Beispielen näher erläutert:

Beispiele:

I. Bestimmungsmethoden:

I.1 Die Partikelgrößenverteilung der nanoskaligen Füllstoffe der Gesamtmenge (B1) wurde mittels dynamischer Lichtstreuung an einem ZetaSizer Nano ZS der Firma Malvern bestimmt. Dazu wurden die zu untersuchenden Nanopartikel in einem geeigneten Lösungsmittel mit Hilfe von Ultraschall fein dispergiert und anschließend bei einer Wellenlänge von 633 nm vermessen. Ausgewertet und angezeigt wurden die erhaltenen Daten zur volumengewichteten Partikelgrößenverteilung mit der ZetaSizer Software V7.13. Die angegebenen Partikelgrößen sind die d50-Werte bei volumengewichteter Auswertung.

I.2 Die Partikelgrößenverteilung der mikroskaligen Füllstoffe der Gesamtmenge (B2) wurde mittels statischer Lichtstreuung an einem Beckman Coulter LS 13 320 bestimmt. Die angegebenen Partikelgrößen sind die d50-Werte bei volumengewichteter Auswertung.

I.3 Die Transluzenz wurde an hergestellten Probekörpern gehärteten Dentalmaterials bestimmt, indem die dentale lichthärtbare Zusammensetzung in eine ringförmige Metallform mit einem Innendurchmesser von 12 mm und einer Dicke von 2 mm eingebracht, oben und unten mit einer Polyesterfolie abgedeckt, verpresst, lichtgehärtet und anschließend entformt wurde, wobei die so hergestellten Probekörper gehärteten Dentalmaterials mit einem Zweistrahl-Spektralphotometer ColorFlex EZ 45/0 der Firma Hunterlab unter Normlicht D65 und unter einem 10° Beobachterwinkel im Reflektionsmodus gegen eine schwarze und eine weiße Kachel untersucht wurden, um die Tristimulus-Werte $Y_{schwarz}$ und $Y_{weiß}$ aus der Messung gegen die schwarze respektive weiße Kachel zu bestimmen. Hierbei wurde die Transluzenz in Prozent mit der folgenden Formel bestimmt:

$$\text{Transluzenz [\%]} = 100 - 100 \cdot Y_{schwarz}/Y_{weiß}$$

I.4 Die Farbe wurde an hergestellten Probekörpern gehärteten Dentalmaterials bestimmt, indem die dentale lichthärtbare Zusammensetzung in eine ringförmige Metallform mit einem Innendurchmesser von 12 mm und einer Dicke von 2 mm eingebracht, oben und unten mit einer Polyesterfolie abgedeckt, verpresst, lichtgehärtet und anschließend entformt wurde, wobei die so hergestellten Probekörper gehärteten Dentalmaterials mit einem Zweistrahl-Spektralphotometer ColorFlex EZ 45/0 der Firma Hunterlab unter Norm-licht D65 und unter einem 10° Beobachterwinkel im Reflektionsmodus gegen eine schwarze und eine weiße Kachel untersucht wurden. Die L*a*b*-Werte entstammen der Messung gegen die weiße Kachel.

I.5 Die Brechungsindizes der Gesamtmenge (A) an radikalisch polymerisierbaren Monomeren wurden bei 20 °C mit einem automatischen Dispersions-Refraktometer Schmidt + Haensch ATR-L gemessen und für die Wellenlängen 470 nm (blau) und 589 nm (gelbe Natrium-D-Linie) notiert.

I.6 Die Brechungsindizes der Sole (kolloidale Lösung von nicht-aggregierten und nicht-agglomerierten anorganischen Füllstoffpartikeln mit einer Partikelgröße im Bereich von 7 bis 70 nm der Gesamtmenge (B) in der Gesamtmenge (A) an radikalisch polymerisierbaren Monomeren) wurden bei 20 °C mit einem automatischem Dispersions-Refraktometer Schmidt + Haensch ATR-L gemessen und für die Wellenlängen 470 nm (blau) und 589 nm (gelbe Natrium-D-Linie) notiert. Über die Gleichung $n_{Sol} = x_{B1} \times n_{B1} + x_A \times n_A$ mit den Brechungsindizes n und den volumenprozentualen Anteilen x wurde auf den Brechungsindex $n_{B1}$ der Gesamtmenge (B1) an nicht-aggregierten und nicht-agglomerierten anorganischen Füllstoffpartikeln mit einer Partikelgröße im Bereich von 7 bis 70 nm (Nanopartikel) zurückgerechnet.

I.7 Die Bestimmung der Brechungsindizes für die Wellenlängen 470 nm (blau) und 589 nm (gelbe Natrium-D-Linie) der Gesamtmenge (B2) an anorganischen Füllstoffpartikeln mit einer Partikelgröße im Bereich von 0,12 $\mu$m bis 10 $\mu$m (Mikropartikel) erfolgte nach der Immersionsmethode. Die Mikropartikel wurden bei 20 °C in Flüssigkeiten mit unterschiedlichen Brechungsindizes dispergiert (sogenannte Immersionsflüssigkeiten). Dabei zeigen sich die Konturen der Mikropartikel umso deutlicher, je größer der Brechzahlunterschied zwischen Flüssigkeit und Mikropartikel ist. Ändert man nun die Brechzahl der Flüssigkeit so, dass sie der der Mikropartikel näherkommt, werden die Partikelkonturen schwächer und verschwinden bei Angleichung der Brechindizes völlig. Als Immersionsflüssigkeiten eignen sich Flüssigkeiten mit bekanntem Brechungsindex, z.B. Mischungen aus Benzylsalicylat (nD20 = 1,536) und Triacetin (nD20 = 1,431) oder Bromnaphthalin (nD20 = 1,657). Durch die Variation der Mengenverhältnisse dieser Stoffe kann der Brechungsindex der Mischung an den des zu messenden Mikropartikels angepasst werden. Bei Übereinstimmung der Brechungsindizes wird der Brechungsindex der Immersionsflüssigkeit wie oben beschrieben mit einem Refraktometer bestimmt. Die Übereinstimmung der Brechungsindizes von Mikropartikel und Immersionsflüssigkeit kann durch Betrachtung der Becke'schen Linie ermittelt werden (Becke-Linien-Verfahren). Dabei handelt es sich um einen hellen Lichtsaum, der sich beim Defokussieren einer Grenzfläche zeigt. Der zu prüfende Mikropartikel wird in eine Flüssigkeit mit bekanntem Brechungsindex gegeben und im Mikroskop mit monochromatischem Licht der entsprechenden Wellenlängen 470 nm (blau) und 589 nm (gelbe Natrium-D-Linie) beobachtet. Besitzen Partikel und Flüssigkeit unterschiedliche Brechungsindizes, zeigt sich um jeden Partikel ein schmaler, leuchtender Ring (Becke-Linie), der sich beim Fokussieren bewegt. Dieser Vorgang wird in verschiedenen Flüssigkeiten mit anderen Brechungsindizes so lange wiederholt, bis keine Becke-Linien mehr auftreten und somit die Brechungsindizes von Partikel und Flüssigkeit übereinstimmen.

I.8 Die Durchhärtetiefe (DHT) (= Polymerisationstiefe) wurde nach dem in ISO 4049:2019 unter Polymerisationstiefe beschriebenen Verfahren mit einem Lichthärtegerät Celalux 2 (VOCO GmbH, Cuxhaven) ermittelt. Die Messungen wurden bei Einstrahlung von Licht aus der LED des Celalux-Lichthärtegeräts, mit einem Strahlungsflussmaximum bei einer Wellenlänge im Bereich von 440 bis 490 nm, einer Bestrahlungsdauer von 10 s bzw. 20 s und einer Bestrahlungsintensität von 1000 mW/cm$^2$ durchgeführt.

I.9 Der Elastizitätsmodul (E-Modul) wurde aus einem Spannungs-Dehnungs-Diagramm einer Biegefestigkeitsmessung anhand der Steigung im linear-elastischen Bereich des Graphen bestimmt. Die Bestimmung der Biegefestigkeit erfolgte durch Herstellung und Aushärtung von Prüfkörpern mit den Dimensionen 2×2×25 mm analog zu dem in der ISO 4049:2019 beschriebenen Verfahren. Die Messung, bei der die Prüfkörper in einem Dreipunktbiegeversuch bis zum Bruch belastet werden, erfolgte an einer Universalprüfmaschine entsprechend der Vorgaben aus der Norm, ebenso die Auswertung.

I.10 Die ACTA-Abrasion wurde nach Aushärtung mittels einer Verschleißsimulation bestimmt. Die Verschleißsimulation erfolgte mit einer Dreimedienabrasionsmaschine TMA 1 (SD-Mechatronik, 83620 Feldkirchen-Westerham) nach de Gee (de Gee, A.J.; Pallav, P.; Occlusal wear simulation with the ACTA wear machine; J. Dent.; 1994; 22; Suppl. 1; S. 21-27) und ISO/TS 14569-2. Die Auswertung erfolgte mit einem Laserscanner TMAM LS (SD-Mechatronik, Feldkirchen-Westerham).

I.11 Die Polymerisationsschrumpfung wurde nach der bonded-disc-Methode (Watts, D. C.; Cash, A. J.; Detemination of polymerization shrinkage kinetics in visible-lightcured materials: methods development; Dent. Mater.; 1991; 7; 281) ermittelt. Die Prüfkörper wurden bei 23 °C für 40 s mit dem Lichthärtegerät Celalux 2 (VOCO GmbH, Cuxhaven) belichtet. Die Schrumpfung wurde nach einer Stunde abgelesen.

II. Verwendete Materialien:

Die in den Beispielen eingesetzten Materialien sind in Tabelle 1 aufgelistet. Die eingesetzten Materialien sind dabei lediglich exemplarisch. Auch andere Materialien und Materialkombinationen können verwendet werden, soweit sie Eigenschaften besitzen, wie sie in erfindungsgemäßen dentalen lichthärtbaren Zusammensetzungen vorliegen müssen; zu den entsprechenden Eigenschaften derjeweiligen Materialien vgl. die vorstehende Beschreibung und die Patentansprüche.

Die Auflistung in Tabelle 1 nennt die chemische Bezeichnung, die im folgenden verwendeten Abkürzungen sowie die Handelsbezeichnung und die CAS Nummer (CAS Nr.) der verwendeten Materialien. Des Weiteren werden die Materialien in Spalte 5 der Tabelle 1 ("Beispiel für") den definierten Gesamtmengen (A), (B1), (B2), (C), (D) und (E) gemäß vorstehender Beschreibung und den nachfolgenden Ansprüchen zugeordnet.

In Tabelle 1 bedeuten:

(1) radikalisch polymerisierbares Monomer der Gesamtmenge (A), wobei diese Gesamtmenge aus ein, zwei, drei oder mehr als drei radikalisch polymerisierbaren Monomeren besteht und einen Brechungsindex $n_{A, 470nm}$ im Bereich von 1,470 bis 1,560 besitzt

(2) nicht-aggregierte und nicht-agglomerierte anorganische Füllstoffpartikel der Gesamtmenge (B1) mit einer Partikelgröße im Bereich von 7 bis 70 nm, wobei diese Gesamtmenge einen Brechungsindex $n_{B1, 470nm}$ im Bereich von 1,460 bis 1,570 besitzt

(3) Oberflächenmodifizierer der Füllstoffpartikel der Gesamtmenge (B1)

(4) anorganische Füllstoffpartikel der Gesamtmenge (B2) mit einer Partikelgröße im Bereich von 0,12 $\mu$m bis 10 $\mu$m, wobei diese Gesamtmenge einen Brechungsindex $n_{B2, 470nm}$ im Bereich von 1,450 bis 1,560 besitzt

(5) Farbpigmente der Gesamtmenge (D) an Farbmitteln

(6) Photoinitiatoren der Gesamtmenge (C)

(7) Hilfsstoffe der Gesamtmenge (E)

**Tabelle 1**

| Bezeichnung | Abkürzung | Handelsbezeichnung | CAS Nr. | Beispiel für |
|---|---|---|---|---|
| Bisphenol-A-glycidyldimethacrylat | Bis-GMA | CN151 | 1565-94-2 | (1) |
| Tricyclodecandimethanol Dimethacrylat | TCD-DMA | SR 834 | 43048-08-4 | (1) |
| ethoxyliertes Bisphenol A-dimethacrylat | Bis-EMA | Miramer M245 | 41637-38-1 | (1) |
| Urethandimethacrylat | UDMA | HEMA-TMDI | 72869-86-4 | (1) |

(fortgesetzt)

| Bezeichnung | Abkürzung | Handelsbezeichnung | CAS Nr. | Beispiel für |
|---|---|---|---|---|
| 2,2'-Ethylendioxydiethyldimethacrylat | TEDMA | SR 205 | 109-16-0 | (1) |
| Ytterbiumtrifluorid Nanopartikel, 50 nm | - | - | 13760-80-0 | (2) |
| Ytterbiumtrifluorid Nanopartikel, 20 nm | - | - | 13760-80-0 | (2) |
| Ytterbiumtrifluorid Nanopartikel, 15 nm | - | - | 13760-80-0 | (2) |
| Siliciumoxid Nanopartikel, 50nm | - | - | 7631-86-9 | (2) |
| Mit MDP oberflächenbeschichtete Ytterbiumtrifluorid Nanopartikel, 50 nm | 50 nm $YbF_3$ + MDP | - | - | (2) |
| Mit MDP oberflächenbeschichtete Ytterbiumtrifluorid Nanopartikel, 20 nm | 20 nm $YbF_3$ + MDP | - | - | (2) |
| Mit MDP oberflächenbeschichtete Ytterbiumtrifluorid Nanopartikel, 15 nm | 15 nm $YbF_3$ + MDP | - | - | (2) |
| Mit MPS oberflächenbeschichtete Siliciumoxid Nanopartikel, 50 nm | 50 nm SiOz + MPS | - | - | (2) |
| 10-Methacryloyloxydecyl Dihydrogen Phosphat | MDP | - | 85590-00-7 | (3) |
| Methacryloxypropyltrimethoxysilan | MPS | A 174 | 2530-85-0 | (3) |
| Bariumaluminiumborosilikatglass, $0,7\mu m$ | Glaskeramik $0,7\mu m$ | GM27884 UF0,7 sil | 65997-17-3 | (4) |
| Bariumaluminiumborosilikatglass, $2,0\mu m$ | Glaskeramik $2,0\mu m$ | GM27884 UF2,0 sil | 65997-17-3 | (4) |
| Quarzglas 0,7 $\mu m$ | - | G018-066 | | (4) |
| Eisenoxid gelb | FS gelb | C.I. Pigment Yellow 42 | 51274-00-1 | (5) |
| Eisenoxid rot | FS rot | C.I. Pigment Red 101 | 1309-37-1 | (5) |
| Eisenoxid schwarz | FS schwarz | C.I. Pigment Black 11 | 1317-61-9 | (5) |
| Titaniumdioxid | FS weiß | C.I. Pigment White 6 | 13463-67-7 | (5) |
| Campherchinon | CQ | DL-Bornane-2,3-dione | 10373-78-1 | (6) |
| Ethyl-4-dimethylamino-benzoat | DABE | Irgacure EDB | 10287-53-3 | (6) |
| 3,5-Di-tert-butyl-4-hydrox-ytoluene (Polymerisationsinhibitor) | BHT | Topanol | 128-37-0 | (7) |
| 2-(2*H*-Benzotriazol-2-yl)-4-methyl-phenol (UV-Stabilisator) | BZT | Drometrizole | 2440-22-4 | (7) |

III. Herstellung von dentalen lichthärtbaren Zusammensetzungen (Beispiele 1-15):

In einem ersten Schritt wurden für die Herstellung der einzelnen dentalen lichthärtbaren Zusammensetzungen (Beispiele 1-15) die jeweiligen radikalisch polymerisierbaren Monomere unter Rühren ineinander gelöst. Es resultierte eine jeweilige Gesamtmenge (A) an radikalisch polymerisierbaren Monomeren, von der anschließend der Brechungsindex $n_{A, 470nm}$ bzw. $n_{A, 589nm}$ bestimmt wurde (vgl. die Messmethode zur Bestimmung der Brechungsindizes).

In einem zweiten Schritt wurde die jeweilige Gesamtmenge (B1) an nicht-aggregierten und nicht-agglomerierten anorganischen Füllstoffpartikeln mit einer Partikelgröße im Bereich von 7 bis 70 nm (die als kolloidale Lösung in Alkohol vorliegenden Ytterbiumtrifluorid bzw. Siliciumoxid Nanopartikel) zusammen mit der im ersten Schritt hergestellten Gesamtmenge (A) in einen Kolben eingewogen, die resultierende Mischung durch Rühren homogenisiert und am Rotationsverdampfer vom Lösungsmittel befreit. Anschließend wurde der Brechungsindex des Sols, d. h. der kolloidalen Lösung von nicht-aggregierten und nicht-agglomerierten anorganischen Füllstoffpartikeln mit einer Partikelgröße im Bereich von 7 bis 70 nm der Gesamtmenge (B1) in der Gesamtmenge (A) an radikalisch polymerisierbaren Monomeren, bestimmt (vgl. die Messmethode zur Bestimmung der Brechungsindizes).

In einem dritten Schritt wurden die jeweiligen Photoinitiatoren der Gesamtmenge (C), die jeweiligen Farbmittel der Gesamtmenge (D) und die jeweiligen Hilfsstoffe der Gesamtmenge (E) in die homogenisierte und vom Lösungsmittel befreite Mischung, die nach Schritt 2 resultierte, unter Rühren eingetragen.

In einem vierten Schritt wurde die jeweilige Gesamtmenge (B2) an anorganischen Füllstoffpartikeln mit einer Partikelgröße im Bereich von 0,12 $\mu$m bis 10 $\mu$m mittels eines Mischgerätes AM 501 der Firma Hauschild in die nach dem dritten Schritt resultierende Mischung eingearbeitet und anschließend zu einer homogenen pastösen Masse verarbeitet (4*12 s), die für 10 min bei 50 U/min und -0,85 bar in einem Laborkneter mit Balkenrührern (PC Laborsystem, Magden, CH) entlüftet wurde.

IV. Zusammensetzungen der Beispiele:

IV.1 Erfindungsgemäße Beispiele:

Die folgenden Tabellen beschreiben exemplarisch die Zusammensetzungen von erfindungsgemäßen dentalen lichthärtbaren Zusammensetzungen, zum wahlweisen Herstellen einer sichtbaren Restauration im Frontzahnbereich oder zum Füllen einer Kavität im Seitenzahnbereich. Sämtliche Abkürzungen entsprechen den zugehörigen Materialien aus der Tabelle 1.

Die verwendeten Mischungsverhältnisse, die eingesetzten Materialien, d.h. die radikalisch polymerisierbaren Monomere der Gesamtmenge (A), die nicht-aggregierten und nicht-agglomerierten anorganischen Füllstoffpartikel mit einer Partikelgröße im Bereich von 7 bis 70 nm der Gesamtmenge (B1), die anorganischen Füllstoffpartikel mit einer Partikelgröße im Bereich von 0,12 $\mu$m bis 10 $\mu$m der Gesamtmenge (B2), die Photoinitiatoren der Gesamtmenge (C), die Farbmittel der Gesamtmenge (D) und die Hilfsstoffe der Gesamtmenge (E) sind dabei lediglich exemplarisch und auch andere Konzentrationen, Materialien und Materialkombinationen können verwendet werden; zu den entsprechenden Eigenschaften vgl. die vorstehende Beschreibung.

Die Beispiele sind unterteilt in "ungefärbte" Beispielrezepturen und "gefärbte" Beispielrezepturen, vgl. die jeweiligen Bezeichnungen in der ersten Zeile der nachfolgenden Tabellen. Ungefärbte Beispielrezepturen enthalten keine Farbmittel der Gesamtmenge (D). Gefärbte Beispielrezepturen enthalten Farbmittel der Gesamtmenge (D).

Die folgenden Tabellen geben zudem die Brechungsindizes der jeweiligen Gesamtmengen (A), (B1) und (B2) für die Wellenlängen 470 nm (spezifisches blaues Licht) und 589 nm (spezifisches gelbes Licht, Natrium-D-Linie) an. Die Brechungsindizes wurden dabei anhand der oben beschriebenen Messmethoden bestimmt. In Klammern werden zudem die Brechungsindex-Differenzen zwischen den Gesamtmengen (A) und (B1) ($\Delta$n (A), (B1)) sowie zwischen den Gesamtmengen (A) und (B2) ($\Delta$n (A), (B2)) angeben; auch hier jeweils bestimmt bei den Wellenlängen 470 nm und 589 nm. Die Brechungsindex-Differenzen zwischen den Gesamtmengen (B1) und (B2) sind in separaten Zeilen angegeben; auch hier jeweils bestimmt bei den Wellenlängen 470 nm ($\Delta$ $n_{470\,nm}$ (B1), $n_{470\,nm}$ (B2)) und 589 nm ($\Delta$ $n_{589\,nm}$ (B1), $n_{589\,nm}$ (B2)).

**Beispiel 1 (erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| **(A)** | Bis-GMA | 2,55% | 5,88% | 2,54% | 5,86% |
| | TCD-DMA | 0,44% | 1,09% | 0,44% | 1,09% |
| | Bis-EMA | 10,63% | 25,82% | 10,62% | 25,79% |
| | $n_{A, 470nm}$ | 1,55430 | | | |
| | $n_{A, 589nm}$ | 1,54017 | | | |
| **(B1)** | 20 nm $YbF_3$ + MDP | 29,79% | 11,98% | 29,82% | 12,01% |
| | $n_{B1, 470nm}$ ($\Delta n$ (A), (B1)) | 1,54870 (0,00560) | | | |
| | $n_{B1, 589nm}$ ($\Delta n$ (A), (B1)) | 1,54216 (-0,00199) | | | |
| **(B2)** | Glaskeramik 0,7 $\mu$m | 9,57% | 9,30% | 9,57% | 9,30% |
| | Glaskeramik 2,0 $\mu$m | 46,80% | 45,45% | 46,76% | 45,44% |
| | $n_{B2, 470nm}$ ($\Delta n$ (A), (B2)) | 1,53570 (0,01860) | | | |
| | $n_{B2, 589nm}$ (An (A), (B2)) | 1,52800 (0,01217) | | | |
| | $\Delta n_{470\,nm}$ (B1), $n_{470\,nm}$ (B2) | 0,01300 | | | |
| | $\Delta n_{589\,nm}$ (B1), $n_{589\,nm}$ (B2) | 0,01416 | | | |
| **(C)** | Campherchinon | 0,04% | 0,09% | 0,04% | 0,09% |
| | DABE | 0,06% | 0,14% | 0,06% | 0,14% |
| **(D)** | FS schwarz | - | - | 0,003% | 0,002% |
| | FS weiß | - | - | 0,01% | 0,006% |
| | FS gelb | - | - | 0,03% | 0,017% |
| | FS rot | - | - | 0,004% | 0,002% |
| **(E)** | BHT | 0,04% | 0,10% | 0,04% | 0,10% |
| | BZT | 0,07% | 0,13% | 0,07% | 0,13% |

**Beispiel 2 (erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| **(A)** | Bis-GMA | 2,68% | 7,39% | 2,68% | 7,38% |
| | TCD-DMA | 0,28% | 0,83% | 0,28% | 0,82% |
| | Bis-EMA | 5,63% | 16,37% | 5,63% | 16,37% |
| | $n_{A, 470nm}$ | 1,55600 | | | |
| | $n_{A, 589nm}$ | 1,54187 | | | |
| **(B1)** | 50 nm $YbF_3$ + MDP | 45,93% | 22,32% | 45,92% | 22,32% |
| | $n_{B1, 470nm}$ ($\Delta n$ (A), (B1)) | 1,54863 (0,00737) | | | |
| | $n_{B1, 589nm}$ ($\Delta n$ (A), (B1)) | 1,54209 (-0,00022) | | | |

(fortgesetzt)

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (B2) | Glaskeramik 0,7 $\mu$m | 7,56% | 8,79% | 7,56% | 8,79% |
| | Glaskeramik 2,0 $\mu$m | 37,78% | 43,93% | 37,78% | 43,93% |
| | $n_{B2, 470nm}$ ($\Delta n$ (A), (B2)) | 1,53570 (0,02030) | | | |
| | $n_{B2, 589nm}$ ($\Delta n$ (A), (B2)) | 1,52800 (0,01387) | | | |
| | $\Delta n_{470\,nm}$ (B1), $n_{470\,nm}$ (B2) | 0,01293 | | | |
| | $\Delta n_{589\,nm}$ (B1), $n_{589\,nm}$ (B2) | 0,01409 | | | |
| (C) | Campherchinon | 0,02% | 0,07% | 0,03% | 0,07% |
| | DABE | 0,04% | 0,11% | 0,04% | 0,11% |
| (D) | FS schwarz | - | - | 0,002% | 0,002% |
| | FS gelb | - | - | 0,02% | 0,014% |
| | FS rot | - | - | 0,003% | 0,002% |
| (E) | BHT | 0,03% | 0,08% | 0,03% | 0,08% |
| | BZT | 0,04% | 0,10% | 0,04% | 0,10% |

**Beispiel 3 (erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (A) | Bis-GMA | 3,07% | 8,56% | 3,06% | 8,55% |
| | Bis-EMA | 6,13% | 18,09% | 6,13% | 18,08% |
| | $n_{A, 470nm}$ | 1,55600 | | | |
| | $n_{A, 589nm}$ | 1,54187 | | | |
| (B1) | 50 nm YbF$_3$ + MDP | 49,35% | 24,27% | 49,34% | 24,27% |
| | $n_{B1, 470nm}$ ($\Delta n$ (A), (B1)) | 1,54863 (0,00737) | | | |
| | $n_{B1, 589nm}$ ($\Delta n$ (A), (B1)) | 1,54209 (-0,00022) | | | |
| (B2) | Glaskeramik 0,7 $\mu$m | 41,28% | 48,66% | 41,26% | 48,66% |
| | $n_{B2, 470nm}$ ($\Delta n$ (A), (B2)) | 1,53570 (0,02030) | | | |
| | $n_{B2, 589nm}$ ($\Delta n$ (A), (B2)) | 1,52800 (0,01387) | | | |
| | $\Delta n_{470\,nm}$ (B1), $n_{470\,nm}$ (B2) | 0,01293 | | | |
| | $\Delta n_{589\,nm}$ (B1), $n_{589\,nm}$ (B2) | 0,01409 | | | |
| (C) | Campherchinon | 0,03% | 0,08% | 0,03% | 0,08% |
| | DABE | 0,04% | 0,12% | 0,04% | 0,12% |
| (D) | FS schwarz | - | - | 0,003% | 0,002% |
| | FS gelb | - | - | 0,02% | 0,017% |
| | FS rot | - | - | 0,004% | 0,002% |

(fortgesetzt)

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (E) | BHT | 0,04% | 0,09% | 0,04% | 0,09% |
| | BZT | 0,06% | 0,12% | 0,07% | 0,12% |

**Beispiel 4 (erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (A) | Bis-GMA | 2,92% | 8,00% | 2,91% | 7,97% |
| | TCD-DMA | 5,14% | 15,10% | 5,12% | 15,05% |
| | Bis-EMA | 0,26% | 0,74% | 0,28% | 0,81% |
| | $n_{A,\,470nm}$ | 1,52987 | | | |
| | $n_{A,\,589nm}$ | 1,51860 | | | |
| (B1) | 50 nm $YbF_3$ + MDP | 44,50% | 21,47% | 44,48% | 21,47% |
| | $n_{B1,\,470nm}$ ($\Delta n$ (A), (B1)) | 1,54863 (-0,01876) | | | |
| | $n_{B1,\,589nm}$ ($\Delta n$ (A), (B1)) | 1,54209 (-0,02349) | | | |
| (B2) | Glaskeramik 0,7 $\mu$m | 7,84% | 9,06% | 7,84% | 9,05% |
| | Glaskeramik 2,0 $\mu$m | 39,21% | 45,28% | 39,19% | 45,26% |
| | $n_{B2,\,470nm}$ ($\Delta n$ (A), (B2)) | 1,53570 (-0,00583) | | | |
| | $n_{B2,\,589nm}$ ($\Delta n$ (A), (B2)) | 1,52800 (-0,00940) | | | |
| | $\Delta$ $n_{470\,nm}$ (B1), $n_{470\,nm}$ (B2) | 0,01293 | | | |
| | $\Delta$ $n_{589\,nm}$ (B1), $n_{589\,nm}$ (B2) | 0,01409 | | | |
| (C) | Campherchinon | 0,02% | 0,07% | 0,02% | 0,07% |
| | DABE | 0,04% | 0,10% | 0,03% | 0,10% |
| (D) | FS schwarz | - | - | 0,004% | 0,003% |
| | FS weiß | - | - | 0,014% | 0,010% |
| | FS gelb | - | - | 0,02% | 0,015% |
| | FS rot | - | - | 0,004% | 0,002% |
| (E) | BHT | 0,03% | 0,08% | 0,03% | 0,08% |
| | BZT | 0,04% | 0,10% | 0,04% | 0,10% |

**Beispiel 5 (erfindungsgemäß, ungefärbt und eingefärbt):**

| Gesamtmenge | Materialien / Brechungsindizes | Ungefärbt | | Eingefärbt | |
| --- | --- | --- | --- | --- | --- |
| | | [Gew-%] | [Vol-%] | [Gew-%] | [Vol-%] |
| (A) | Bis-GMA | 2,83% | 7,79% | 2,82% | 7,76% |
| | TCD-DMA | 2,78% | 8,20% | 2,77% | 8,17% |
| | Bis-EMA | 2,93% | 8,50% | 2,94% | 8,54% |
| | $n_{A,\,470nm}$ | 1,54315 | | | |
| | $n_{A,\,589nm}$ | 1,53044 | | | |
| (B1) | 50 nm $YbF_3$ + MDP | 45,64% | 22,13% | 45,62% | 22,13% |
| | $n_{B1,\,470nm}$ ($\Delta n$ (A), (B1)) | 1,54863 (-0,00548) | | | |
| | $n_{B1,\,589nm}$ ($\Delta n$ (A), (B1)) | 1,54209 (-0,01165) | | | |
| (B2) | Glaskeramik 0,7 $\mu m$ | 7,62% | 8,84% | 7,61% | 8,84% |
| | Glaskeramik 2,0 $\mu m$ | 38,08% | 44,18% | 38,06% | 44,17% |
| | $n_{B2,\,470nm}$ ($\Delta n$ (A), (B2)) | 1,53570 (0,00745) | | | |
| | $n_{B2,\,589nm}$ ($\Delta n$ (A), (B2)) | 1,52800 (0,00244) | | | |
| | $\Delta n_{470\,nm}$ (B1), $n_{470\,nm}$ (B2) | 0,01293 | | | |
| | $\Delta n_{589\,nm}$ (B1), $n_{589\,nm}$ (B2) | 0,01409 | | | |
| (C) | Campherchinon | 0,02% | 0,07% | 0,02% | 0,07% |
| | DABE | 0,04% | 0,11% | 0,04% | 0,10% |
| (D) | FS schwarz | - | - | 0,004% | 0,003% |
| | FS weiß | - | - | 0,009% | 0,007% |
| | FS gelb | - | - | 0,02% | 0,017% |
| | FS rot | - | - | 0,004% | 0,0024% |
| (E) | BHT | 0,03% | 0,08% | 0,03% | 0,08% |
| | BZT | 0,04% | 0,10% | 0,04% | 0,10% |

**Beispiel 6 (erfindungsgemäß, ungefärbt und eingefärbt):**

| Gesamtmenge | Materialien / Brechungsindizes | Ungefärbt | | Eingefärbt | |
| --- | --- | --- | --- | --- | --- |
| | | [Gew-%] | [Vol-%] | [Gew-%] | [Vol-%] |
| (A) | Bis-GMA | 5,36% | 14,83% | 5,35% | 14,80% |
| | TCD-DMA | 0,28% | 0,83% | 0,28% | 0,82% |
| | Bis-EMA | 2,95% | 8,60% | 2,96% | 8,63% |
| | $n_{A,\,470nm}$ | 1,56034 | | | |
| | $n_{A,\,589nm}$ | 1,54610 | | | |
| (B1) | 50 nm $YbF_3$ + MDP | 45,92% | 22,41% | 45,91% | 22,41% |
| | $n_{B1,\,470nm}$ ($\Delta n$ (A), (B1)) | 1,54863 (0,01171) | | | |
| | $n_{B1,\,589nm}$ ($\Delta n$ (A), (B1)) | 1,54209 (0,00401) | | | |

(fortgesetzt)

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (B2) | Glaskeramik 0,7 μm | 7,58% | 8,85% | 7,58% | 8,85% |
| | Glaskeramik 2,0 μm | 37,78% | 44,11% | 37,77% | 44,10% |
| | $n_{B2, 470nm}$ ($\Delta n$ (A), (B2)) | 1,53570 (0,02464) | | | |
| | $n_{B2, 589nm}$ ($\Delta n$ (A), (B2)) | 1,52800 (0,01810) | | | |
| | $\Delta n_{470 nm}$ (B1), $n_{470 nm}$ (B2) | 0,01293 | | | |
| | $\Delta n_{589 nm}$ (B1), $n_{589 nm}$ (B2) | 0,01409 | | | |
| (C) | Campherchinon | 0,02% | 0,07% | 0,02% | 0,07% |
| | DABE | 0,04% | 0,11% | 0,04% | 0,11% |
| (D) | FS schwarz | - | - | 0,003% | 0,002% |
| | FS gelb | - | - | 0,02% | 0,014% |
| | FS rot | - | - | 0,003% | 0,0020% |
| (E) | BHT | 0,03% | 0,08% | 0,03% | 0,08% |
| | BZT | 0,04% | 0,10% | 0,04% | 0,10% |

**Beispiel 7 (erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (A) | Bis-GMA | 2,53% | 5,79% | 2,52% | 5,77% |
| | TCD-DMA | 0,44% | 1,07% | 0,44% | 1,07% |
| | Bis-EMA | 10,53% | 25,41 % | 10,51% | 25,39% |
| | $n_{A, 470nm}$ | 1,55430 | | | |
| | $n_{A, 589nm}$ | 1,54017 | | | |
| (B1) | 15 nm $YbF_3$ + MDP | 29,77% | 12,66% | 29,81% | 12,68% |
| | $n_{B1, 470nm}$ ($\Delta n$ (A), (B1)) | 1,54347 (0,01083) | | | |
| | $n_{B1, 589nm}$ ($\Delta n$ (A), (B1)) | 1,53684 (0,00333) | | | |
| (B2) | Glaskeramik 0,7 μm | 9,42% | 9,10% | 9,41% | 9,10% |
| | Glaskeramik 2,0 μm | 47,11% | 45,49% | 47,07% | 45,48% |
| | $n_{B2, 470nm}$ ($\Delta n$ (A), (B2)) | 1,53570 (0,01860) | | | |
| | $n_{B2, 589nm}$ ($\Delta n$ (A), (B2)) | 1,52800 (0,01217) | | | |
| | $\Delta n_{470 nm}$ (B1), $n_{470 nm}$ (B2) | 0,00777 | | | |
| | $\Delta n_{589 nm}$ (B1), $n_{589 nm}$ (B2) | 0,00884 | | | |
| (C) | Campherchinon | 0,04% | 0,09% | 0,04% | 0,09% |
| | DABE | 0,06% | 0,14% | 0,06% | 0,14% |

(fortgesetzt)

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| **(D)** | FS schwarz | - | - | 0,005% | 0,002% |
| | FS weiß | - | - | 0,008% | 0,005% |
| | FS gelb | - | - | 0,02% | 0,014% |
| | FS rot | - | - | 0,004% | 0,0020% |
| **(E)** | BHT | 0,04% | 0,10% | 0,04% | 0,10% |
| | BZT | 0,07% | 0,13% | 0,07% | 0,13% |

**Beispiel 8 (erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| **(A)** | Bis-GMA | 0,95% | 2,61% | 0,95% | 2,60% |
| | TCD-DMA | 0,27% | 0,80% | 0,27% | 0,80% |
| | Bis-EMA | 7,22% | 20,90% | 7,22% | 20,89% |
| | $n_{A,\,470nm}$ | 1,55284 | | | |
| | $n_{A,\,589nm}$ | 1,53876 | | | |
| **(B1)** | 50 nm $YbF_3$ + MDP | 45,20% | 21,86% | 45,19% | 21,86% |
| | $n_{B1,\,470nm}$ ($\Delta n$ (A), (B1)) | 1,54863 (0,004219 | | | |
| | $n_{B1,\,589nm}$ ($\Delta n$ (A), (B1)) | 1,54209 (-0,00333) | | | |
| **(B2)** | Glaskeramik 0,7 $\mu m$ | 7,70% | 8,91% | 7,70% | 8,91% |
| | Glaskeramik 2,0 $\mu m$ | 38,51% | 44,56% | 38,51% | 44,56% |
| | $n_{B2,\,470nm}$ ($\Delta n$ (A), (B2)) | 1,53570 (0,01714) | | | |
| | $n_{B2,\,589nm}$ ($\Delta n$ (A), (B2)) | 1,52800 (0,01076) | | | |
| | $\Delta\,n_{470\,nm}$ (B1), $n_{470\,nm}$ (B2) | 0,01293 | | | |
| | $\Delta\,n_{589\,nm}$ (B1), $n_{589\,nm}$ (B2) | 0,01409 | | | |
| **(C)** | Campherchinon | 0,02% | 0,07% | 0,02% | 0,07% |
| | DABE | 0,04% | 0,11% | 0,04% | 0,11% |
| **(D)** | FS schwarz | - | - | 0,003% | 0,002% |
| | FS gelb | - | - | 0,02% | 0,013% |
| | FS rot | - | - | 0,003% | 0,0020% |
| **(E)** | BHT | 0,03% | 0,08% | 0,03% | 0,08% |
| | BZT | 0,04% | 0,10% | 0,04% | 0,10% |

**Beispiel 9 (erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (A) | UDMA | 8,61% | 14,06% | 8,60% | 14,05% |
| | TEDMA | 9,83% | 16,50% | 9,82% | 16,49% |
| | $n_{A, 470nm}$ | 1,48123 | | | |
| | $n_{A, 589nm}$ | 1,47224 | | | |
| (B1) | 50 nm SiO2 + MPS | 28,44 | 25,56% | 28,44% | 25,56% |
| | $n_{B1, 470nm}$ ($\Delta n$ (A), (B1)) | 1,46660 (0,01463) | | | |
| | $n_{B1, 589nm}$ ($\Delta n$ (A), (B1)) | 1,46070 (0,01154) | | | |
| | Quarzglas 0,7 $\mu$m | 52,79% | 43,40% | 52,78% | 43,40% |
| | $n_{B2, 470nm}$ ($\Delta n$ (A), (B2)) | 1,46410 (0,01713) | | | |
| | $n_{B2, 589nm}$ ($\Delta n$ (A), (B2)) | 1,45840 (0,01384) | | | |
| | $\Delta n_{470\,nm}$ (B1), $n_{470\,nm}$ (B2) | 0,00250 | | | |
| | $\Delta n_{589\,nm}$ (B1), $n_{589\,nm}$ (B2) | 0,00230 | | | |
| (C) | Campherchinon | 0,05% | 0,09% | 0,05% | 0,09% |
| | DABE | 0,08% | 0,13% | 0,08% | 0,13% |
| (D) | FS schwarz | - | - | 0,003% | 0,001% |
| | FS weiß | - | - | 0,007% | 0,01% |
| | FS gelb | - | - | 0,02% | 0,014% |
| | FS rot | - | - | 0,004% | 0,002% |
| (E) | BHT | 0,07% | 0,11% | 0,07% | 0,11% |
| | BZT | 0,12% | 0,14% | 0,12% | 0,14% |

IV.2 Nicht-erfindungsgemäße Beispiele:

Die folgenden Tabellen betreffen exemplarisch die Zusammensetzungen von nicht-erfindungsgemäßen dentalen lichthärtbaren Zusammensetzung.

**Beispiel 10 (nicht-erfindungsgemäß, ungefärbt und eingefärbt):**

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| (A) | Bis-GMA | 4,17% | 7,77% | 4,16% | 7,74% |
| | TCD-DMA | 0,43% | 0,86% | 0,43% | 0,86% |
| | Bis-EMA | 8,76% | 17,19% | 8,72% | 17,13% |
| | $n_{A, 470nm}$ | 1,55600 | | | |
| | $n_{A, 589nm}$ | 1,54187 | | | |
| (B1) | 50 nm $SiO_2$ + MPS | 20,52% | 22,05% | 20,61% | 22,15% |
| | $n_{B1, 470nm}$ ($\Delta n$ (A), (B1)) | 1,46660 (0,08940) | | | |
| | $n_{B1, 589nm}$ ($\Delta n$ (A), (B1)) | 1,46070 (0,08117) | | | |

(fortgesetzt)

| Gesamtmenge | Materialien / Brechungsindizes | Ungefärbt | | Eingefärbt | |
|---|---|---|---|---|---|
| | | [Gew-%] | [Vol-%] | [Gew-%] | [Vol-%] |
| **(B2)** | Glaskeramik 0,7 μm | 12,01% | 9,43% | 11,99% | 9,42% |
| | Glaskeramik 2,0 μm | 53,91% | 42,32% | 53,83% | 42,29% |
| | $n_{B2, 470nm}$ ($\Delta n$ (A), (B2)) | 1,53570 (0,02030) | | | |
| | $n_{B2, 589nm}$ ($\Delta n$ (A), (B2)) | 1,52800 (0,01387) | | | |
| | $\Delta n_{470\,nm}$ (B1), $n_{470\,nm}$ (B2) | -0,06910 | | | |
| | $\Delta n_{589\,nm}$ (B1), $n_{589\,nm}$ (B2) | -0,06730 | | | |
| **(C)** | Campherchinon | 0,04% | 0,07% | 0,04% | 0,08% |
| | DABE | 0,06% | 0,11% | 0,06% | 0,11% |
| **(D)** | FS schwarz | - | - | 0,003% | 0,001% |
| | FS weiß | - | - | 0,01% | 0,01% |
| | FS gelb | - | - | 0,03% | 0,018% |
| | FS rot | - | - | 0,005% | 0,002% |
| **(E)** | BHT | 0,04% | 0,08% | 0,04% | 0,08% |
| | BZT | 0,07% | 0,11% | 0,07% | 0,10% |

Die dentalen Zusammensetzungen gemäß Beispiel 10 (ungefärbt bzw. eingefärbt) besitzen gute mechanische Eigenschaften; die Brechungsindizes der eingesetzten

Materialien sind jedoch nicht in erfindungsgemäßer Weise aufeinander abgestimmt. Einzelheiten hierzu ergeben sich aus dem nachfolgenden Kapitel IV.3. Konsequenzen hinsichtlich der Eigenschaften Durchhärtetiefe und Transluzenz ergeben sich aus Kapitel V.1.

**Beispiele 11 und 12 (nicht-erfindungsgemäß, ungefärbt):**

| Gesamtmenge | Materialien / Brechungsindizes | Beispiel 11 | | Beispiel 12 | |
|---|---|---|---|---|---|
| | | [Gew-%] | [Vol-%] | [Gew-%] | [Vol-%] |
| **(A)** | Bis-GMA | 6,53% | 12,60% | 5,28% | 11,34% |
| | TCD-DMA | 0,68% | 1,40% | 0,55% | 1,26% |
| | Bis-EMA | 13,70% | 27,89% | 11,09% | 25,10% |
| | $n_{A, 470nm}$ | 1,55600 | | 1,55600 | |
| | $n_{A, 589nm}$ | 1,54187 | | 1,54187 | |
| **(B1)** | 50 nm $YbF_3$ + MDP | 13,94% | 4,70% | 24,92% | 9,34% |
| | $n_{B1, 470nm}$ ($\Delta n$ (A), (B1)) | 1,54863 (0,00737) | | 1,54863 (0,00737) | |
| | $n_{B1, 589nm}$ ($\Delta n$ (A), (B1)) | 1,54209 (-0,00022) | | 1,54209 (-0,00022) | |

(fortgesetzt)

| Gesamtmenge | Materialien / Brechungsindizes | Beispiel 11 | | Beispiel 12 | |
|---|---|---|---|---|---|
| | | [Gew-%] | [Vol-%] | [Gew-%] | [Vol-%] |
| **(B2)** | Glaskeramik 0,7 $\mu$m | 10,81% | 8,80% | 9,65% | 8,74% |
| | Glaskeramik 2,0 $\mu$m | 54,05% | 44,02% | 48,26% | 43,69% |
| | $n_{B2, 470nm}$ ($\Delta$n (A), (B2)) | 1,53570 (0,02030) | | 1,53570 (0,02030) | |
| | $n_{B2, 589nm}$ ($\Delta$n (A), (B2)) | 1,52800 (0,01387) | | 1,52800 (0,01387) | |
| | $\Delta$ $n_{470 nm}$ (B1), $n_{470 nm}$ (B2) | 0,01293 | | 0,01293 | |
| | $\Delta$ $n_{589 nm}$ (B1), $n_{589 nm}$ (B2) | 0,01409 | | 0,01409 | |
| **(C)** | Campherchinon | 0,06% | 0,12% | 0,05% | 0,11% |
| | DABE | 0,09% | 0,18% | 0,07% | 0,17% |
| **(E)** | BHT | 0,06% | 0,13% | 0,05% | 0,11% |
| | BZT | 0,10% | 0,16% | 0,08% | 0,14% |

**Beispiele 13-14 (nicht-erfindungsgemäß, ungefärbt):**

| Gesamtmenge | Materialien / Brechungsindizes | Beispiel 13 | | Beispiel 14 | |
|---|---|---|---|---|---|
| | | [Gew-%] | [Vol-%] | [Gew-%] | [Vol-%] |
| **(A)** | Bis-GMA | 3,55% | 10,32% | 3,91% | 11,64% |
| | TCD-DMA | 0,37% | 1,15% | 0,41% | 1,30% |
| | Bis-EMA | 7,45% | 22,84% | 8,20% | 25,76% |
| | $n_{A, 470nm}$ | 1,55600 | | 1 ,55600 | |
| | $n_{A, 589nm}$ | 1,54187 | | 1,54187 | |
| **(B1)** | 50 nm $YbF_3$ + MDP | 60,72% | 31,14% | 66,91% | 35,13% |
| | $n_{B1, 470nm}$ ($\Delta$n (A), (B1)) | 1,54863 (0,00737) | | 1,54863 (0,00737) | |
| | $n_{B1, 589nm}$ ($\Delta$n (A), (B1)) | 1,54209 (-0,00022) | | 1,54209 (-0,00022) | |
| **(B2)** | Glaskeramik 0,7 $\mu$m | 4,62% | 5,67% | 3,40% | 4,27% |
| | Glaskeramik 2,0 $\mu$m | 23,12% | 28,37% | 16,98% | 21,33% |
| | $n_{B2, 470nm}$ ($\Delta$n (A), (B2)) | 1,53570 (0,02030) | | 1,53570 (0,02030) | |
| | $n_{B2, 589nm}$ ($\Delta$n (A), (B2)) | 1,52800 (0,01387) | | 1,52800 (0,01387) | |
| | $\Delta$ $n_{470 nm}$ (B1), $n_{470 nm}$ (B2) | 0,01293 | | 0,01293 | |
| | $\Delta$ $n_{589 nm}$ (B1), $n_{589 nm}$ (B2) | 0,01409 | | 0,01409 | |
| **(C)** | Campherchinon | 0,03% | 0,10% | 0,04% | 0,11% |
| | DABE | 0,05% | 0,15% | 0,05% | 0,17% |
| **(E)** | BHT | 0,03% | 0,11% | 0,04% | 0,12% |
| | BZT | 0,06% | 0,14% | 0,06% | 0,16% |

**Beispiel 15 (nicht-erfindungsgemäß, ungefärbt):**

| Gesamtmenge | Materialien / Brechungsindizes | Ungefärbt | |
|---|---|---|---|
| | | [Gew-%] | [Vol-%] |
| (A) | Bis-GMA | 7,86% | 13,58% |
| | TCD-DMA | 0,81% | 1,51% |
| | Bis-EMA | 16,51% | 30,05% |
| | $n_{A,\,470nm}$ | 1,55600 | |
| | $n_{A,\,589nm}$ | 1,54187 | |
| (B2) | Glaskeramik 0,7 $\mu$m | 12,41% | 9,03% |
| | Glaskeramik 2,0 $\mu$m | 62,02% | 45,16% |
| | $n_{B2,\,470nm}$ ($\triangle$n (A), (B2)) | 1,53570 (0,02030) | |
| | $n_{B2,\,589nm}$ ($\triangle$n (A), (B2)) | 1,52800 (0,01387) | |
| (C) | Campherchinon | 0,07% | 0,13% |
| | DABE | 0,11% | 0,20% |
| (E) | BHT | 0,08% | 0,14% |
| | BZT | 0,13% | 0,18% |

IV.3 Unterscheidung der Beispiele:

Die dentalen lichthärtbaren Zusammensetzungen (Beispiele 1-15) unterscheiden sich wie folgt:
Die erfindungsgemäßen dentalen lichthärtbaren Zusammensetzungen 1-9 umfassen eine Gesamtmenge (A) an radikalisch polymerisierbaren Monomeren, eine Gesamtmenge (B1) an nicht-aggregierten und nicht-agglomerierten anorganischen Füllstoffpartikeln mit einer Partikelgröße im Bereich von 7 bis 70 nm und eine Gesamtmenge (B2) an anorganischen Füllstoffpartikeln mit einer Partikelgröße im Bereich von 0,12 $\mu$m bis 10 $\mu$m.
In den erfindungsgemäßen dentalen lichthärtbaren Zusammensetzungen 1-9 sind die Materialien und die Mischungsverhältnisse der

- radikalisch polymerisierbaren Monomere der jeweiligen Gesamtmengen (A) so ausgewählt und aufeinander abgestimmt, dass die Gesamtmenge (A) jeweils einen Brechungsindex $n_{A,\,470nm}$ im Bereich von 1,470 bis 1,560 besitzt (vgl. die entsprechenden Tabelleneinträge $n_{A,\,470nm}$ der erfindungsgemäßen dentalen lichthärtbaren Dentalmaterialien 1-9).

- nicht-aggregierten und nicht-agglomerierten anorganischen Füllstoffpartikel mit einer Partikelgröße im Bereich von 7 bis 70 nm der jeweiligen Gesamtmengen (B1) so ausgewählt und aufeinander abgestimmt, dass die Gesamtmenge (B1) jeweils einen Brechungsindex $n_{B1,\,470nm}$ im Bereich von 1,460 bis 1,570 besitzt (vgl. die entsprechenden Tabelleneinträge $n_{B1,\,470nm}$ der erfindungsgemäßen dentalen lichthärtbaren Dentalmaterialien 1-9).

- anorganischen Füllstoffpartikel mit einer Partikelgröße im Bereich von 0,12 $\mu$m bis 10 $\mu$m der jeweiligen Gesamtmengen (B2) so ausgewählt und aufeinander abgestimmt, dass die Gesamtmenge (B2) jeweils einen Brechungsindex $n_{B2,\,470nm}$ im Bereich von 1,450 bis 1,560 besitzt (vgl. die entsprechenden Tabelleneinträge $n_{B2,\,470nm}$ der erfindungsgemäßen dentalen lichthärtbaren Dentalmaterialien 1-9).

In den erfindungsgemäßen dentalen lichthärtbaren Zusammensetzungen 1-9 gilt zudem, dass die Brechungsindizes der jeweiligen Gesamtmenge (A) an radikalisch polymerisierbaren Monomeren und der jeweiligen Gesamtmenge (B1) an nicht-aggregierten und nicht-agglomerierten anorganischen Füllstoffpartikeln mit einer Partikelgröße im Bereich von 7 bis 70 nm so aufeinander abgestimmt sind, dass folgende Bedingung erfüllt wird:

$$n_{B1,\,470nm} - 0,025 < n_{A,\,470nm} < n_{B1,\,470nm} + 0,030$$

(vgl. die entsprechenden Tabelleneinträge $\triangle$n (A), (B1) der erfindungsgemäßen dentalen lichthärtbaren Zusammensetzungen 1-9).

Des Weiteren gilt in den erfindungsgemäßen dentalen lichthärtbaren Zusammensetzungen 1-9, dass die Brechungsindizes der jeweiligen Gesamtmenge (A) an radikalisch polymerisierbaren Monomeren und der jeweiligen Gesamtmenge (B2) an anorganischen Füllstoffpartikeln mit einer Partikelgröße im Bereich von 0,12 μm bis 10 μm so aufeinander abgestimmt sind, dass folgende Bedingung erfüllt wird:

$$n_{B2, 470nm} - 0{,}015 < n_{A, 470nm} < n_{B2, 470nm} + 0{,}040$$

(vgl. die entsprechenden Tabelleneinträge Δn (A), (B2) der erfindungsgemäßen dentalen lichthärtbaren Zusammensetzungen 1-9).

Des Weiteren gilt in den erfindungsgemäßen dentalen lichthärtbaren Zusammensetzungen 1-9, dass die Brechungsindizes der jeweiligen Gesamtmenge (B1) an nicht-aggregierten und nicht-agglomerierten anorganischen Füllstoffpartikeln mit einer Partikelgröße im Bereich von 7 bis 70 nm und der jeweiligen Gesamtmenge (B2) an anorganischen Füllstoffpartikeln mit einer Partikelgröße im Bereich von 0,12 μm bis 10 μm so aufeinander abgestimmt sind, dass folgende Bedingung erfüllt wird:

$$n_{B2, 470nm} - 0{,}020 < n_{B1, 470nm} < n_{B2, 470nm} + 0{,}035$$

(vgl. die entsprechenden Tabelleneinträge Δ $n_{470\,nm}$ (B1), $n_{470\,nm}$ (B2) der erfindungsgemäßen dentalen lichthärtbaren Zusammensetzungen 1-9).

Die erfindungsgemäßen dentalen lichthärtbaren Zusammensetzungen 1-9 umfassen eine Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) im Bereich von 10 bis 30 Volumenprozent liegt, bezogen auf die jeweilige gesamte dentale lichthärtbare Zusammensetzung (vgl. die die entsprechenden Tabelleneinträge in der Spalte "[Vol-%]" zur Zeile "(B1)" der erfindungsgemäßen dentalen lichthärtbaren Zusammensetzungen 1-9).

Die erfindungsgemäßen dentalen lichthärtbaren Zusammensetzungen 1-9 umfassen eine Gesamtmenge (B1) an nicht-aggregierten und nicht-agglomerierten anorganischen Füllstoffpartikeln mit einer Partikelgröße im Bereich von 7 bis 70 nm. Diese Füllstoffpartikel unterscheiden sich hinsichtlich ihrer Partikelgrößen wie folgt (die angegebenen Partikelgrößen sind die d50-Werte bei volumengewichteter Auswertung, vgl. hierzu auch die Angaben zur Messmethode):

Beispiel 1: $YbF_3$ + MDP; 20 nm

Beispiele 2-6 und 8: $YbF_3$ + MDP; 50 nm

Beispiel 7: $YbF_3$ + MDP; 15 nm

Beispiel 9: $SiO_2$ + MPS; 50 nm

Die erfindungsgemäßen dentalen lichthärtbaren Zusammensetzungen 1-9 umfassen zudem eine Gesamtmenge (C) an Photoinitiatoren (Campherchinon und DABE), eine Gesamtmenge (D) an Farbmitteln (eingefärbte Beispiele 1-9) oder keine Gesamtmenge (D) an Farbmitteln (ungefärbte Beispiele 1-9) sowie eine Gesamtmenge (E) an Hilfsstoffen (BHT als Inhibitor und BZT als UV-Stabilisator).

Das nicht-erfindungsgemäße Beispiel 10 (gefärbt und ungefärbt) umfasst eine Gesamtmenge (A) an radikalisch polymerisierbaren Monomeren und eine Gesamtmenge (B1) an nicht-aggregierten und nicht-agglomerierten anorganischen Füllstoffpartikeln mit einer Partikelgröße im Bereich von 7 bis 70 nm ($SiO_2$ + MPS; 50 nm), wobei der Brechungsindex der Gesamtmenge (A) gemessen bei einer Wellenlänge von 470 nm 1,55600 beträgt, der Brechungsindex der Gesamtmenge (B1) ebenfalls gemessen bei einer Wellenlänge von 470 nm 1,46660 beträgt ($\Delta n_{470nm}$ (A), (B1) = 0,08940) und der Brechungsindex der Gesamtmenge (B2) ebenfalls gemessen bei einer Wellenlänge von 470 nm 1,53570 beträgt ($\Delta n_{470\,nm}$ (B1), $n_{470\,nm}$ (B2) = - 0,06910). Somit werden in Beispiel 10 die folgenden Bedingungen nicht erfüllt:

$$n_{B1, 470nm} - 0{,}025 < n_{A, 470nm} < n_{B1, 470nm} + 0{,}030$$

und

$$n_{B2,\,470nm} - 0{,}020 < n_{B1,\,470nm} < n_{B2,\,470nm} + 0{,}035.$$

Die nicht-erfindungsgemäßen Beispiele 11 und 12 umfassen eine Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) unter 10 Volumenprozent liegt, bezogen auf die gesamte dentale lichthärtbare Zusammensetzung (vgl. die entsprechenden Tabelleneinträge in der Spalte "[Vol-%]" zur Zeile "(B1)" der nicht-erfindungsgemäßen dentalen lichthärtbaren Zusammensetzungen 11 und 12).

Die nicht-erfindungsgemäßen Beispiele 13 und 14 umfassen eine Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) über 30 Volumenprozent liegt, bezogen auf die gesamte dentale lichthärtbare Zusammensetzung (vgl. die entsprechenden Tabelleneinträge in der Spalte "[Vol-%]" zur Zeile "(B1)" der nicht-erfindungsgemäßen dentalen lichthärtbaren Zusammensetzungen 13 und 14).

Das nicht-erfindungsgemäße Beispiel 15 umfasst keine Gesamtmenge (B1) an nicht-aggregierten und nicht-agglomerierten anorganischen Füllstoffpartikeln mit einer Partikelgröße im Bereich von 7 bis 70 nm.

V. <u>Eigenschaften der Beispiele:</u>

V.1 <u>Durchhärtetiefe und Transluzenz:</u>

Die Durchhärtetiefe und die Transluzenz von erfindungsgemäßen dentalen lichthärtbaren Zusammensetzungen werden insbesondere von den Brechungsindizes der darin enthaltenen radikalisch polymerisierbaren Monomere ($n_{A,\,470nm}$ der Gesamtmenge (A)) sowie den Brechungsindizes der Füllstoffpartikel ($n_{B1,\,470nm}$ der Gesamtmenge (B1) und $n_{B2,\,470nm}$ der Gesamtmenge (B2)) bestimmt. Verglichen werden hier nachfolgend die erfindungsgemäßen Beispiele 1-9 mit dem nicht-erfindungsgemä-ßen Beispiel 10, welches Materialien (Gesamtmengen (A), (B1) und (B2)) umfasst, die hinsichtlich ihrer Brechungsindizes nicht in erfindungsgemäßer Weise aufeinander abgestimmt sind.

Die nachfolgende Tabelle 2 zeigt:

- die Durchhärtetiefen (Polymerisationstiefen) bei Einstrahlung von Licht aus einer LED (aus dem Gerät Celalux 2), mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$ (DHT 10 s (1000 mW/cm$^2$) [mm]),

- die Durchhärtetiefen bei Einstrahlung von Licht aus einer LED, mit einem Strahlungsflussmaximum bei einer Wellenlänge im Bereich von 440 bis 490 nm, einer Bestrahlungsdauer von 20 s und einer Bestrahlungsintensität von 1000 mW/cm$^2$ (DHT 20 s (1000 mW/cm$^2$) [mm]),

- die Transluzenz in % (bestimmt nach Lichthärtung) und

- die Farbwerte L*a*b*

der erfindungsgemäßen dentalen lichthärtbaren Zusammensetzungen (Beispiele 1-9) sowie einer nicht-erfindungsgemäßen dentalen lichthärtbaren Zusammensetzung (Beispiel 10). Die in Tabelle 2 aufgeführten Messwerte wurden dabei mittels der vorangehend beschriebenen Messmethoden bestimmt. Soweit keine Messungen durchgeführt wurden, ist in den Feldern der Tabellen jeweils ein Strich "-" eingetragen.

**Tabelle 2**

| Beispiel | | DHT 10 s (1000 mW/cm²) [mm] | DHT 20 s (1000 mW/cm²) [mm] | Transluzenz [%] | L* | a* | b* |
|---|---|---|---|---|---|---|---|
| 1 | ungefärbt | ≥ 6,00 | - | 32,8 | - | - | - |
| | gefärbt | 3,75 | 4,07 | 20,1 | 68,31 | 3,34 | 15,02 |
| 2 | ungefärbt | 5,21 | - | 28,5 | - | - | - |
| | gefärbt | 3,96 | 4,36 | 20,5 | 65,88 | 3,38 | 15,31 |
| 3 | ungefärbt | ≥ 6,00 | - | 28,1 | - | - | - |
| | gefärbt | 3,80 | 4,17 | 18,2 | 68,29 | 3,11 | 15,47 |
| 4 | ungefärbt | ≥ 6,00 | - | 41,4 | - | - | - |
| | gefärbt | 3,57 | 4,19 | 20,2 | 65,86 | 3,58 | 14,95 |
| 5 | ungefärbt | 5,93 | - | 32,2 | - | - | - |
| | gefärbt | 3,78 | 4,24 | 18,5 | 64,4 | 3,23 | 14,12 |
| 6 | ungefärbt | 5,45 | - | 27,4 | - | - | - |
| | gefärbt | 4,57 | 5,05 | 19,8 | 65,95 | 3,26 | 14,53 |
| 7 | ungefärbt | ≥ 6,00 | - | 32,5 | - | - | - |
| | gefärbt | 3,91 | 4,30 | 20,3 | 65,15 | 3,21 | 13,32 |
| 8 | ungefärbt | 5,34 | - | 27,8 | - | - | - |
| | gefärbt | 4,30 | 4,72 | 21,4 | 65,04 | 3,33 | 15,42 |
| 9 | ungefärbt | 5,15 | - | 29,9 | - | - | - |
| | gefärbt | 3,61 | 3,97 | 18,4 | 68,15 | 3,01 | 15,94 |
| 10 (n.e.) | ungefärbt | 4,23 | - | 33,6 | - | - | - |
| | gefärbt | 2,88 | - | 18,7 | 66,52 | 3,94 | 16,61 |

n.e. = nicht-erfindungsgemäß (wegen nicht aufeinander abgestimmter Brechungsindizes der Bestandteile)

Es ergibt sich aus obenstehender Tabelle 2, dass die erfindungsgemäßen dentalen lichthärtbaren Zusammensetzungen der Beispiele 1-9 (gefärbt und ungefärbt) folgende Bedingungen erfüllen:

1.) eine Durchhärtetiefe von 3,5 mm oder mehr, bei Einstrahlung von Licht aus einer LED (aus dem Gerät Celalux 2), mit einem Strahlungsflussmaximum bei einer Wellenlänge im Bereich von 440 bis 490 nm, einer Bestrahlungsdauer von 10 s und einer Bestrahlungsintensität von 1000 mW/cm², sowie

2.) eine Transluzenz von weniger als 45 % (nach Lichthärtung).

Die nicht-erfindungsgemäße dentale lichthärtbare Zusammensetzung des Beispiels 10 (gefärbt) hingegen besitzt eine Durchhärtetiefe, bestimmt bei Einstrahlung von Licht aus einer LED (aus dem Gerät Celalux 2), mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$ (DHT 10 s), von lediglich 2,88 mm.

Für Beispiel 10 (ungefärbt) ergibt sich zwar eine DHT 10 s von 4,23 mm. Da in der dentalen Praxis jedoch insbesondere bei Einsatz einer dentalen lichthärtbaren Zusammensetzung im Frontzahnbereich ausschließlich eingefärbte Zusammensetzungen Verwendung finden, zeigt dieser Wert bereits, dass die ungefärbte dentale lichthärtbare Zusammensetzung des Beispiels 10 als Basiszusammensetzung für den Praxisgebrauch kaum tauglich ist, da bei sinnvoller Einfärbung ein Produkt mit einer zu niedrigen DHT 10 s resultieren würde.

Der ermittelte Wert von 4,23 mm für Beispiel 10 (ungefärbt) ist deutlich niedriger als der ermittelte Wert von 5,15 mm für Beispiel 9 (ungefärbt). Beispiel 9 ist das erfindungsgemäße Beispiel mit dem geringsten Wert DHT 10 s innerhalb der Gruppe der Beispiele 1-9 (jeweils ungefärbt). Somit zeigt Tabelle 2 eindrucksvoll, dass Beispiel 10 (ungefärbt) aufgrund der nicht aneinander angepassten Brechungsindizes der Gesamtmengen (A) zu (B1), d.h. die Ungleichung $n_{B1,\,470nm}$ - 0,025 < $n_{A,\,470nm}$ < $n_{B1,\,470nm}$ + 0,030 wird nicht erfüllt, sowie der ebenfalls nicht aneinander angepassten Brechungsindizes der Gesamtmengen (B1) zu (B2), die Ungleichung $n_{B2,\,470nm}$ - 0,020 < $n_{B1,\,470nm}$ < $n_{B2,\,470nm}$ + 0,035 wird ebenfalls nicht erfüllt, im Unterschied zu den erfindungsgemäßen Beispielen 1-9 (ungefärbt) nicht als Basiszusammensetzung für eingefärbte Zusammensetzungen zum Füllen einer Kavität im Seitenzahnbereich geeignet ist.

Die Durchhärtetiefen der ungefärbten erfindungsgemäßen dentalen lichthärtbaren Zusammensetzungen der Beispiele 1-9, bestimmt bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$, betragen 5,15 mm oder mehr, die ungefärbten erfindungsgemä-ßen Beispiele 1, 3, 4 und 7 weisen sogar Durchhärtetiefen von ≥ 6,00 mm auf.

V.2 <u>Mechanische Eigenschaften:</u>

Eine dentale lichthärtbare Zusammensetzung zum Herstellen einer sichtbaren Restauration im Frontzahnbereich und zum Füllen einer Kavität im Seitenzahnbereich sollte vorzugsweise jeweils hervorragende physikalische und mechanische Eigenschaften aufweisen, wie sie insbesondere durch die Parameter E-Modul, ACTA-Abrasion und Polymerisationsschrumpfung beschrieben werden.

Der Vergleich der mechanischen Eigenschaften der erfindungsgemäßen Beispielzusammensetzungen 1-9 (enthaltend Nanopartikel der Gesamtmenge (B1) in einem Bereich von 10 bis 30 Volumenprozent, bezogen auf die gesamte dentale lichthärtbare Zusammensetzung) mit den nicht-erfindungsgemäßen Beispielzusammensetzungen 11-15 (11 und 12: Gesamtmenge (B1) < 10 Volumenprozent; 13 und 14: Gesamtmenge (B1) > 30 Volumenprozent; 15: ohne Nanopartikel) bestätigt, dass lichthärtbare Zusammensetzungen mit einem prozentualen Anteil von Nanopartikeln von weniger als 10 Volumenprozent bzw. von mehr als 30 Volumenprozent sowie Zusammensetzungen ohne Nanopartikel aufgrund suboptimaler mechanischer Eigenschaften als moderne Dentalkomposite kaum geeignet sind. Diese Zusammensetzungen entsprechen insbesondere nicht den heutigen Anforderungen an die mechanischen Eigenschaften von dentalen lichthärtbaren Zusammensetzung, die zum wahlweisen Herstellen einer sichtbaren Restauration im Frontzahnbereich bzw. zum Füllen einer Kavität im Seitenzahnbereich geeignet sind.

Die nachfolgende Tabelle 3 zeigt den E-Modul [GPa], die ACTA-Abrasion [μm] und die Polymerisationsschrumpfung [%] der erfindungsgemäßen Beispielzusammensetzungen 1-9 sowie der nicht-erfindungsgemäßen Beispielzusammensetzungen 11-15. Die in Tabelle 3 aufgeführten Eigenschaften wurden dabei mittels der oben beschriebenen Messmethoden bestimmt.

**Tabelle 3**

| Beispiel | E-Modul [GPa] | ACTA-Abrasion [μm] | Polymerisationsschrumpfung [%] |
|----------|---------------|--------------------|--------------------------------|
| 1 | 11,0 | 65 | 1,7 |
| 2 | 14,5 | 48 | 1,3 |
| 3 | 12,3 | 58 | 1,4 |
| 4 | 13,2 | 49 | 1,4 |
| 5 | 13,5 | 47 | 1,3 |
| 6 | 13,7 | 45 | 1,1 |
| 7 | 10,7 | 66 | 1,7 |

(fortgesetzt)

| Beispiel | E-Modul [GPa] | ACTA-Abrasion [$\mu$m] | Polymerisationsschrumpfung [%] |
|---|---|---|---|
| 8 | 13,6 | 45 | 1,4 |
| 9 | 10,7 | 59 | 1,7 |
| 11 (n.e.) | 9,8 | 83 | 1,9 |
| 12 (n.e.) | 10,5 | 76 | 1,8 |
| 13 (n.e.) | 8,2 | 85 | 3,0 |
| 14 (n.e.) | 7,3 | 98 | 3,7 |
| 15 (n.e.) | 9,7 | 83 | 2,0 |
| n.e. = nicht-erfindungsgemäß | | | |

Der Vergleich zeigt, dass die erfindungsgemäßen dentalen lichthärtbaren Zusammensetzungen der Beispiele 1-9 (enthaltend nicht-aggregierte und nicht-agglomerierte anorganische Füllstoffpartikel der Gesamtmenge (B1) mit einer Partikelgröße im Bereich von 7 bis 70 nm, wobei diese Gesamtmenge im Bereich von 10 bis 30 Volumenprozent liegt, bezogen auf die gesamte dentale lichthärtbare Zusammensetzung) bessere mechanische Eigenschaften aufweisen als die nicht-erfindungsgemäßen dentalen lichthärtbaren Zusammensetzungen der Beispiele 11-15 (Gesamtmenge (B1) < 10 Volumenprozent; > 30 Volumenprozent; bzw. ohne Gesamtmenge (B1)).

Die gemessenen Werte des E-Moduls der erfindungsgemäßen Beispielzusammensetzungen 1-9 liegen im Bereich von 10,7 GPa bis 14,5 GPa und sind somit in vorteilhafter Weise höher als die gemessenen Werte des E-Moduls der nicht-erfindungsgemäßen Beispielzusammensetzungen 11-15 (Werte im Bereich von 7,3 GPa bis 10,5 GPa).

Die gemessenen Werte der ACTA-Abrasion der erfindungsgemäßen Beispielzusammensetzungen 1-9 liegen im Bereich von 45 $\mu$m bis 66 $\mu$m und sind somit vorteilhaft geringer als die gemessenen Werte der ACTA-Abrasion von nicht-erfindungsgemäßen Beispielzusammensetzungen 11-15 (Werte im Bereich von 76 $\mu$m bis 98 $\mu$m).

Die gemessenen Werte der Polymerisationsschrumpfung der erfindungsgemäßen Beispielzusammensetzungen liegen im Bereich von 1,1 % bis 1,7 % und sind somit vorteilhaft geringer als die gemessenen Werte der Polymerisationsschrumpfung von nicht-erfindungsgemäßen Beispielzusammensetzungen 11-15 (Werte im Bereich von 1,8 % bis 3,7 %).

Somit zeigt sich, dass die erfindungsgemäßen dentalen lichthärtbaren Zusammensetzungen, zum wahlweisen Herstellen einer sichtbaren Restauration im Frontzahnbereich oder zum Füllen einer Kavität im Seitenzahnbereich, einen vorteilhaften technischen Effekt haben in Bezug auf:

i) eine deutlich höhere Festigkeit der ausgehärteten dentalen lichthärtbaren Zusammensetzung (hoher E-Modul) und

ii) eine deutlich höhere Abriebfestigkeit der ausgehärteten dentalen lichthärtbaren Zusammensetzung (geringe ACTA-Abrasion) und

iii) eine deutlich verringerte Schrumpfung bei Aushärtung (sehr geringe Polymerisationsschrumpfung).

Im Vergleich dazu erfüllt keine der nicht-erfindungsgemäßen Beispielzusammensetzungen die Gesamtanforderungen in Bezug auf die mechanischen Eigenschaften.

**Patentansprüche**

1.  Dentale lichthärtbare Zusammensetzung, zum wahlweisen Herstellen einer sichtbaren Restauration im Frontzahnbereich oder zum Füllen einer Kavität im Seitenzahnbereich, umfassend

    - eine Gesamtmenge (A) an radikalisch polymerisierbaren Monomeren, wobei diese Gesamtmenge (A) aus ein, zwei, drei oder mehr als drei radikalisch polymerisierbaren Monomeren besteht und einen Brechungsindex $n_{A, 470nm}$ im Bereich von 1,470 bis 1,560 besitzt,
    - eine Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) einen Brechungsindex $n_{B1, 470nm}$ im Bereich von 1,460 bis 1,570 besitzt und wobei diese Gesamtmenge (B1) im Bereich von 10 bis 30 Volumenprozent liegt, bezogen auf die gesamte dentale lichthärtbare Zusammensetzung,
- eine Gesamtmenge (B2) an anorganischen Füllstoffpartikeln mit einer Partikelgröße im Bereich von 0,12 μm bis 10 μm, wobei diese Gesamtmenge (B2) einen Brechungsindex $n_{B2, 470nm}$ im Bereich von 1,450 bis 1,560 besitzt,
und
- eine Gesamtmenge (C) an Photoinitiatoren,
wobei für die Brechungsindizes der Gesamtmenge (A) an radikalisch polymerisierbaren Monomeren und der Gesamtmenge (B1) an nicht-aggregierten und nicht-agglomerierten anorganischen Füllstoffpartikeln mit einer Partikelgröße im Bereich von 7 bis 70 nm gilt:

$$n_{B1, 470nm} - 0,025 < n_{A, 470nm} < n_{B1, 470nm} + 0,030$$

und
wobei für die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 μm bis 10 μm gilt:

$$n_{B2, 470nm} - 0,015 < n_{A, 470nm} < n_{B2, 470nm} + 0,040$$

und
wobei für die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 μm bis 10 μm gilt:

$$n_{B2, 470nm} - 0,020 < n_{B1, 470nm} < n_{B2, 470nm} + 0,035.$$

2. Dentale lichthärtbare Zusammensetzung nach Anspruch 1,

wobei die Gesamtmenge (A) an radikalisch polymerisierbaren Monomeren, die Gesamtmenge (B1) an nicht-aggregierten und nicht-agglomerierten anorganischen Füllstoffpartikeln mit einer Partikelgröße im Bereich von 7 bis 70 nm, die Gesamtmenge (B2) an anorganischen Füllstoffpartikeln mit einer Partikelgröße im Bereich von 0,12 μm bis 10 μm und die Gesamtmenge (C) an Photoinitiatoren so ausgewählt ist, dass
die dentale lichthärtbare Zusammensetzung
eine Durchhärtetiefe von 3,5 mm oder mehr besitzt, bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm², 
und/oder
die dentale lichthärtbare Zusammensetzung nach der Härtung
eine Transluzenz von weniger als 45 % besitzt,
bevorzugt eine Transluzenz von weniger als 40 % besitzt,
besonders bevorzugt eine Transluzenz von weniger als 35 % besitzt.

3. Dentale lichthärtbare Zusammensetzung nach einem der vorangehenden Ansprüche, wobei für die Brechungsindizes der Gesamtmenge (A) an radikalisch polymerisierbaren Monomeren und der Gesamtmenge (B1) an nicht-aggregierten und nicht-agglomerierten anorganischen Füllstoffpartikeln mit einer Partikelgröße im Bereich von 7 bis 70 nm gilt:

$$n_{B1, 470nm} - 0,020 < n_{A, 470nm} < n_{B1, 470nm} + 0,025,$$

bevorzugt $n_{B1, 470nm} - 0,015 < n_{A, 470nm} < n_{B1, 470nm} + 0,020$,
und/oder

wobei für die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 μm bis 10

μm gilt:

$$n_{B2,\,470nm} - 0,010 < n_{A,\,470nm} < n_{B2,\,470nm} + 0,035,$$

bevorzugt $n_{B2,\,470nm} - 0,005 < n_{A,\,470nm} < n_{B2,\,470nm} + 0,030$,
und/oder
wobei für die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 μm bis 10 μm gilt:

$$n_{B2,\,470nm} - 0,015 < n_{B1,\,470nm} < n_{B2,\,470nm} + 0,030,$$

bevorzugt $n_{B2,\,470nm} - 0,010 < n_{B1,\,470nm} < n_{B2,\,470nm} + 0,025$.

4. Dentale lichthärtbare Zusammensetzung nach einem der vorangehenden Ansprüche, wobei

- die Gesamtmenge (A) an radikalisch polymerisierbaren Monomeren,

im Bereich von 21 bis 34 Volumenprozent liegt, bezogen auf die gesamte dentale lichthärtbare Zusammensetzung
und/oder
im Bereich von 6 bis 20 Gewichtsprozent liegt, bezogen auf die gesamte dentale lichthärtbare Zusammensetzung

und/oder
- die Gesamtmenge (B1) an nicht-aggregierten und nicht-agglomerierten anorganischen Füllstoffpartikeln mit einer Partikelgröße im Bereich von 7 bis 70 nm, bevorzugt die Gesamtmenge (B1-p1) an nicht-aggregierten und nicht-agglomerierten anorganischen Füllstoffpartikeln mit einer Partikelgröße im Bereich von 7 bis 60 nm, besonders bevorzugt die Gesamtmenge (B1-p2) an nicht-aggregierten und nicht-agglomerierten anorganischen Füllstoffpartikeln mit einer Partikelgröße im Bereich von 7 bis 50 nm,

im Bereich von 13 bis 27 Volumenprozent liegt, bevorzugt im Bereich von 15 bis 25 Volumenprozent, bezogen auf die gesamte dentale lichthärtbare Zusammensetzung
und/oder
im Bereich von 10 bis 66 Gewichtsprozent liegt, bevorzugt im Bereich von 13 bis 63 Gewichtsprozent, besonders bevorzugt im Bereich von 14 bis 60 Gewichtsprozent, bezogen auf die gesamte dentale lichthärtbare Zusammensetzung

und/oder
- die Gesamtmenge (B2) an anorganischen Füllstoffpartikeln mit einer Partikelgröße im Bereich von 0,12 μm bis 10 μm, bevorzugt die Gesamtmenge (B2-p1) an anorganischen Füllstoffpartikeln mit einer Partikelgröße im Bereich von 0,18 μm bis 10 μm, besonders bevorzugt die Gesamtmenge (B2-p2) an anorganischen Füllstoffpartikeln mit einer Partikelgröße im Bereich von 0,40 μm bis 10 μm,

im Bereich von 42 bis 60 Volumenprozent liegt, bezogen auf die gesamte dentale lichthärtbare Zusammensetzung
und/oder
im Bereich von 28 bis 70 Gewichtsprozent liegt, bezogen auf die gesamte dentale lichthärtbare Zusammensetzung,

und/oder
- sich die Gesamtmengen (A), (B1) und (B2) der dentalen lichthärtbaren Zusammensetzung

auf zumindest 95 Volumenprozent, vorzugsweise zumindest 98 Volumenprozent addieren, bezogen auf die gesamte dentale lichthärtbare Zusammensetzung (d. h. deren Gesamtvolumen),
und/oder

auf zumindest 95 Gewichtsprozent, vorzugsweise 98 Gewichtsprozent addieren, bezogen auf die gesamte dentale lichthärtbare Zusammensetzung (d. h. deren Gesamtmasse).

5. Dentale lichthärtbare Zusammensetzung nach einem der vorangehenden Ansprüche, wobei

die Gesamtmenge (B1) an nicht-aggregierten und nicht-agglomerierten anorganischen Füllstoffpartikeln mit einer Partikelgröße im Bereich von 7 bis 70 nm

- aus Füllstoffpartikeln besteht, die (i) eine gleiche oder unterschiedliche stoffliche Zusammensetzung besitzen und (ii) zusätzlich oberflächenmodifiziert sind oder nicht oberflächenmodifiziert sind,

wobei die Füllstoffpartikel vorzugsweise (i) eine gleiche stoffliche Zusammensetzung besitzen und (ii) zusätzlich oberflächenmodifiziert sind,
wobei die Gesamtmenge (B1) vorzugsweise

- aus einer Menge von Füllstoffpartikeln gleicher stofflicher Zusammensetzung besteht, welche einen Brechungsindex $n_{B1,\ 470nm}$ im Bereich von 1,460 bis 1,570 besitzt, wobei in dieser Menge die Füllstoffpartikel zusätzlich oberflächenmodifiziert sind oder nicht oberflächenmodifiziert sind,
oder
- aus einer Mischung von zwei, drei oder mehr als drei Teilmengen von Füllstoffpartikeln besteht, wobei die Füllstoffpartikel innerhalb einer jeden Teilmenge (i) eine gleiche stoffliche Zusammensetzung besitzen und (ii) zusätzlich oberflächenmodifiziert sind oder nicht oberflächenmodifiziert sind, aber die Füllstoffpartikel aus unterschiedlichen Teilmengen eine unterschiedliche stoffliche Zusammensetzung besitzen, wobei jede dieser Teilmengen für sich betrachtet einen Brechungsindex $n_{B1,\ 470nm}$ im Bereich von 1,460 bis 1,570 besitzt, wobei vorzugsweise die Differenz zwischen dem höchsten Brechungsindex und dem niedrigsten Brechungsindex der jeweils für sich betrachteten Teilmengen kleiner ist als 0,01,

und/oder
die Gesamtmenge (B2-p2) an anorganischen Füllstoffpartikeln mit einer Partikelgröße im Bereich von 0,40 $\mu m$ bis 10 $\mu m$, bevorzugt die Gesamtmenge (B2-p1) an anorganischen Füllstoffpartikeln mit einer Partikelgröße im Bereich von 0,18 $\mu m$ bis 10 $\mu m$, besonders bevorzugt die Gesamtmenge (B2) an anorganischen Füllstoffpartikeln mit einer Partikelgröße im Bereich von 0,12 $\mu m$ bis 10 $\mu m$

- aus einer Menge von Füllstoffpartikeln gleicher stofflicher Zusammensetzung besteht, die vorzugsweise zusätzlich oberflächenmodifiziert sind, wobei diese Menge von Füllstoffpartikeln einen Brechungsindex $n_{B2,\ 470nm}$ im Bereich von 1,450 bis 1,560 besitzt,
oder
- aus einer Mischung von zwei, drei oder mehr als drei Teilmengen von Füllstoffpartikeln besteht, wobei die Füllstoffpartikel innerhalb einer jeden Teilmenge eine gleiche stoffliche Zusammensetzung besitzen und vorzugsweise zusätzlich oberflächenmodifiziert sind, aber die Füllstoffpartikel aus unterschiedlichen Teilmengen eine unterschiedliche stoffliche Zusammensetzung besitzen, wobei jede dieser Teilmengen für sich betrachtet einen Brechungsindex $n_{B2,\ 470nm}$ im Bereich von 1,450 bis 1,560 besitzt, wobei vorzugsweise die Differenz zwischen dem höchsten Brechungsindex und dem niedrigsten Brechungsindex der jeweils für sich betrachteten Teilmengen kleiner ist als 0,01.

6. Dentale lichthärtbare Zusammensetzung nach einem der vorangehenden Ansprüche, wobei die Gesamtmenge (A) an radikalisch polymerisierbaren Monomeren so ausgewählt ist, dass durch ihre Lichthärtung ein polymeres Material erhältlich ist, welches einen um mindestens 0,015, vorzugsweise mindestens 0,020, erhöhten Brechungsindex besitzt, bestimmt bei einer Wellenlänge von 589 nm.

7. Dentale lichthärtbare Zusammensetzung nach einem der vorangehenden Ansprüche, zusätzlich zu den Gesamtmengen (A), (B1), (B2) und (C) umfassend eine Gesamtmenge (D) an Farbmitteln ausgewählt aus der Gruppe bestehend aus anorganischen Farbpigmenten, organischen Farbpigmenten und Farbstoffen,

wobei vorzugsweise

- für die Transluzenz $T_{Col}$ dieser dentalen lichthärtbaren Zusammensetzung nach der Härtung im Vergleich mit der Transluzenz $T_{NoCol}$ einer ansonsten identisch zusammengesetzten dentalen lichthärtbaren Zusammensetzung, die keine zusätzlichen Farbmittel umfasst, gilt:
35 % > $T_{NoCol}$ - $T_{Col}$ > 5 %, bevorzugt 30 % > $T_{NoCol}$ - $T_{Col}$ > 5 %, besonders bevorzugt 25 % > $T_{NoCol}$ - $T_{Col}$ > 5 %,

und/oder

- die Gesamtmenge (D) an dem einen oder den mehreren Farbmitteln

maximal einen Volumenanteil von 800 ppm bildet, bevorzugt maximal 600 ppm, besonders bevorzugt maximal 400 ppm, bezogen auf die gesamte dentale lichthärtbare Zusammensetzung, und/oder
maximal einen Massenanteil von 2000 ppm bildet, bevorzugt maximal 1500 ppm, besonders bevorzugt maximal 1000 ppm, bezogen auf die gesamte dentale lichthärtbare Zusammensetzung,

und/oder
- die dentale lichthärtbare Zusammensetzung nach der Härtung
eine Transluzenz $T_{col}$ von weniger als 28 % besitzt,
bevorzugt von weniger als 26 %,
besonders bevorzugt von weniger als 24 %,
insbesondere bevorzugt on weniger als 22 %.

8. Dentale lichthärtbare Zusammensetzung nach einem der vorangehenden Ansprüche, wobei in der Zusammensetzung die Gesamtmenge sämtlicher anorganischer Partikel mit einer Partikelgröße von maximal 0,12 $\mu$m eine durchschnittliche volumenbezogene Partikelgröße von weniger als 70 nm besitzt, bestimmt mittels dynamischer Lichtstreuung, vorzugsweise eine durchschnittliche volumenbezogene Partikelgröße von weniger als 66 nm.

9. Dentale lichthärtbare Zusammensetzung nach einem der vorangehenden Ansprüche, wobei für die Farbwerte der dentalen lichthärtbaren Zusammensetzung nach der Härtung, bestimmt gemäß EN ISO 11664-4, gilt:

55,0 < L* < 80,0, bevorzugt 64,0 < L* < 75,0, und
-1,5 < a* < 4,5, bevorzugt 2,0 < a* < 4,5, und
5,0 < b* < 20,0, bevorzugt 13,0 < b* < 20,0,
wobei bevorzugt gilt:

64,0 < L* < 75,0, und
2,0 < a* < 4,5, und
13,0 < b* < 20,0.

10. Dentale lichthärtbare Zusammensetzung nach einem der vorangehenden Ansprüche, zusätzlich zu den Gesamtmengen (A), (B1), (B2) und (C) umfassend eine Gesamtmenge (E) an ein, zwei, drei oder mehr als drei Hilfsstoffen, bei denen es sich nicht um Farbmittel handelt, wobei die Hilfsstoffe der Gesamtmenge (E) ausgewählt sind aus der Gruppe bestehend aus:

- rheologische Hilfsmittel,
- Polymerisationsinitiatoren, die keine Photoinitiatoren sind,
- chemische Verbindungen als Katalysatoren bzw. Bestandteile von Katalysatorsystemen,
- Stabilisatoren, insbesondere UV- und Tageslichtstabilisatoren,
- Inhibitoren,
- Aktivatoren,
- Molekulargewichtsregler,
- Konservierungsmittel,
- grenzflächenaktive Substanzen,
- Biozide, vorzugsweise Bakterizide,
- organische Polymere und Oligomere und Verbindungen mit hohen Molekulargewichten, vorzugsweise Weichmacher,
- Verdickungsmittel, und
- dentale Arzneimittel.

11. Dentale Restauration oder gehärtetes Dentalmaterial, vorzugsweise sichtbare dentale Restauration im Frontzahnbereich oder Füllung einer Kavität im Seitenzahnbereich, erhältlich durch Lichthärtung einer dentalen lichthärtbaren Zusammensetzung nach einem der Ansprüche 1 bis 10, vorzugsweise unter Verwendung von Licht in einem Wellenlängenbereich von 450 nm bis 490 nm.

12. Verfahren zur Herstellung einer dentalen lichthärtbaren Zusammensetzung, vorzugsweise nach einem der Ansprüche 1 bis 10, umfassend die folgenden Schritte:

(i) Auswahl einer Gesamtmenge (A) an radikalisch polymerisierbaren Monomeren, einer Gesamtmenge (B1) an nicht-aggregierten und nicht-agglomerierten anorganischen Füllstoffpartikeln mit einer Partikelgröße im Bereich von 7 bis 70 nm, einer Gesamtmenge (B2) an anorganischen Füllstoffpartikeln mit einer Partikelgröße im Bereich von 0,12 $\mu$m bis 10 $\mu$m und einer Gesamtmenge (C) an Photoinitiatoren,
wobei folgende Kriterien zur Auswahl der Gesamtmengen (A), (B1) und (B2) gelten:

- die Gesamtmenge (A) an radikalisch polymerisierbaren Monomeren besitzt einen Brechungsindex $n_{A, 470nm}$ im Bereich von 1,470 bis 1,560,
- die Gesamtmenge (B1) an nicht-aggregierten und nicht-agglomerierten anorganischen Füllstoffpartikeln mit einer Partikelgröße im Bereich von 7 bis 70 nm besitzt einen Brechungsindex $n_{B1, 470nm}$ im Bereich von 1,460 bis 1,570,
- die Gesamtmenge (B2) an anorganischen Füllstoffpartikeln mit einer Partikelgröße im Bereich von 0,12 $\mu$m bis 10 $\mu$m besitzt einen Brechungsindex $n_{B2, 470nm}$ im Bereich von 1,450 bis 1,560,
- für die Brechungsindizes der Gesamtmenge (A) an radikalisch polymerisierbaren Monomeren und der Gesamtmenge (B1) an nicht-aggregierten und nicht-agglomerierten anorganischen Füllstoffpartikeln mit einer Partikelgröße im Bereich von 7 bis 70 nm gilt:

$$n_{B1, 470nm} - 0,025 < n_{A, 470nm} < n_{B1, 470nm} + 0,030,$$

- für die Brechungsindizes der Gesamtmenge (A) an radikalisch polymerisierbaren Monomeren und der Gesamtmenge (B2) an anorganischen Füllstoffpartikeln mit einer Partikelgröße im Bereich von 0,12 $\mu$m bis 10 $\mu$m gilt:

$$n_{B2, 470nm} - 0,015 < n_{A, 470nm} < n_{B2, 470nm} + 0,040,$$

- für die Brechungsindizes der Gesamtmenge (B1) an nicht-aggregierten und nicht-agglomerierten anorganischen Füllstoffpartikeln mit einer Partikelgröße im Bereich von 7 bis 70 nm und der Gesamtmenge (B2) an anorganischen Füllstoffpartikeln mit einer Partikelgröße im Bereich von 0,12 $\mu$m bis 10 $\mu$m gilt:

$$n_{B2, 470nm} - 0,020 < n_{B1, 470nm} < n_{B2, 470nm} + 0,035,$$

(ii) Herstellen oder Bereitstellen der ausgewählten Gesamtmengen (A), (B1), (B2) und (C),
(iii) Mischen der gemäß Schritt (ii) hergestellten bzw. bereitgestellten Gesamtmengen (A), (B1), (B2) und (C), sowie gegebenenfalls zusätzlich einer Gesamtmenge (D) von Farbmitteln und/oder einer Gesamtmenge (E) an ein, zwei, drei oder mehr als drei zusätzlichen Hilfsstoffen, so dass die dentale lichthärtbare Zusammensetzung resultiert.

13. Verwendung einer, zweier oder sämtlicher der folgenden Gesamtmengen:

- Gesamtmenge (A) an radikalisch polymerisierbaren Monomeren, wobei diese Gesamtmenge (A) aus ein, zwei, drei oder mehr als drei radikalisch polymerisierbaren Monomeren besteht und einen Brechungsindex $n_{A, 470nm}$ im Bereich von 1,470 bis 1,560 besitzt,
- Gesamtmenge (B1) an nicht-aggregierten und nicht-agglomerierten anorganischen Füllstoffpartikeln mit einer Partikelgröße im Bereich von 7 bis 70 nm, wobei diese Gesamtmenge (B1) einen Brechungsindex $n_{B1, 470nm}$ im Bereich von 1,460 bis 1,570 besitzt,
- Gesamtmenge (B2) an anorganischen Füllstoffpartikeln mit einer Partikelgröße im Bereich von 0,12 $\mu$m bis 10 $\mu$m, wobei diese Gesamtmenge (B2) einen Brechungsindex $n_{B2, 470nm}$ im Bereich von 1,450 bis 1,560 besitzt,

zur Herstellung einer dentalen lichthärtbaren Zusammensetzung, die geeignet ist zum wahlweisen Herstellen einer sichtbaren Restauration im Frontzahnbereich oder zum Füllen einer Kavität im Seitenzahnbereich, wobei die dentale lichthärtbare Zusammensetzung

- eine Durchhärtetiefe von 3,5 mm oder mehr besitzt, bei Einstrahlung von Licht aus einer LED, mit einem Strahlungsflussmaximum bei einer Wellenlänge im Bereich von 440 bis 490 nm, einer Bestrahlungsdauer von 10 s und einer Bestrahlungsintensität von 1000 mW/cm$^2$, und/oder
- nach der Härtung eine Transluzenz von weniger als 45 % besitzt.

14. Dentale lichthärtbare Zusammensetzung nach einem der Ansprüche 1 bis 10 zur Anwendung in einem Verfahren zur chirurgischen oder therapeutischen Behandlung des menschlichen oder tierischen Körpers und/oder zur Anwendung in einem Diagnostizierverfahren, das am menschlichen oder tierischen Körper vorgenommen wird, vorzugsweise zur spezifischen Anwendung

- in einem therapeutischen Verfahren zur temporären oder permanenten sichtbaren Restauration im Frontzahnbereich oder in einem therapeutischen Verfahren zum temporären oder permanenten Füllen einer Kavität im Seitenzahnbereich, oder
- in einem therapeutischen Verfahren als

  - Zahnfüllungsmaterial,
  - Dentalzement,
  - dentales Unterfüllungsmaterial,
  - als fließfähiges Kompositmaterial (Flow-Material),
  - als Kronenmaterial,
  - als Inlay,
  - als Onlay,
  - als Brückenmaterial
  - und/oder als Stumpfaufbaumaterial.

15. Verfahren zur Herstellung einer sichtbaren Restauration im Frontzahnbereich oder zum Füllen einer Kavität im Seitenzahnbereich umfassend die folgenden Schritte:

  (i) Herstellen oder Bereitstellen einer dentalen lichthärtbaren Zusammensetzung nach einem der Ansprüche 1 bis 10,
  (ii) Applizieren der hergestellten oder bereitgestellten dentalen lichthärtbaren Zusammensetzung auf die zu restaurierende oder zu füllende Zahnpartie,
  (iii) Aushärten der applizierten dentalen lichthärtbaren Zusammensetzung durch Bestrahlen mit Licht im Wellenlängenbereich von 450 nm bis 490 nm.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2987480 A1
- EP 2902007 A1
- EP 3508190 A1
- EP 3854374 A1
- WO 2021148667 A1
- CN 113730264 A
- DE 2419887 A1
- DE 2406557 A1
- DE 2931926 A1
- DE 3522005 A1
- DE 3522006 A1
- DE 3703120 A1
- DE 102005021332 A1
- DE 102005053775 A1
- DE 102006060983 A1
- DE 69935794 T2
- DE 102007034457 A1
- EP 0783880 B1
- DE 10119831 A1
- EP 1563821 A1

**In der Beschreibung aufgeführte Nicht-Patentliteratur**

- *CHEMICAL ABSTRACTS,* 85590-00-7
- *CHEMICAL ABSTRACTS,* 2530-85-0
- **DE GEE, A.J. ; PALLAV, P.** Occlusal wear simulation with the ACTA wear machine. *J. Dent.,* 1994, vol. 22 (1), 21-27
- **WATTS, D. C. ; CASH, A. J.** Detemination of polymerization shrinkage kinetics in visible-lightcured materials: methods development. *Dent. Mater.,* 1991, vol. 7, 281